# EUROPEAN PATENT APPLICATION

(11) **EP 2 295 985 A1**
(43) Date of publication of application: **16.03.2011**
(21) Application number: 09794444.1
(22) Date of filing: 07.07.2009
(51) Int. Cl.: G01N 35/00, G01N 37/00

(54) **DISC FOR CLINICAL EXAMINATION, DISC PACK AND CLINICAL EXAMINATION DEVICE**

(30) Priority: 10.07.2008 JP 2008180668; 10.07.2008 JP 2008180669
(71) Applicant: Sanko Junyaku Co., Ltd., Chiyoda-ku Tokyo 101-0032 (JP); Eisai R&D Management Co., Ltd., Tokyo 112-8088 (JP)
(72) Inventor: KAWAMURA, Toshimi, (JP); ISHIOKA, Seiichirou, (JP); TONARI, Masaki, (JP); KAWAMURA, Takashi, (JP); NAKAJIMA, Akira, (JP); SEKINO, Tetsuo, (JP); IWAHASHI, Kyoichi, (JP); UCHIYAMA, Kenichi, (JP); TAKASE, Tomohiro, (JP); NAWATA, Isao, (JP)
(74) Representative: Friedrich, Andreas
(86) International application number: PCT/JP2009/062382
(87) International publication number: WO 2010/005000

(57) **Abstract**

A clinical examination disk according to the invention includes a disk body in a disk form, and at least one cell formed in the disk body, wherein the cell comprises a specimen reservoir formed in the disk body and having a specimen injecting port, a reaction tank formed in the disk body so as to position nearer to the outer circumference of the body than a position of the specimen reservoir, and holding a reagent which is reactive with the specimen and is beforehand gelatinized, a first narrow tube formed in the disk body, and communicating to the specimen reservoir and the reaction tank, and a second narrow tube formed in the disk body, and having one end connected to the reaction tank and the other end made open.

## Description

### Technical Field

The present invention relates to a clinical examination disk, a disk pack, and a clinical examination device.

### Background Art

In an actual clinical examination, collected blood is centrifuged with a centrifugal separator, and then blood plasma, which is a supernatant, is taken out. The taken-out blood plasma is mixed with a reagent in a reaction vessel to be caused to react therewith. The fluorescence therefrom or the absorbance thereof is measured. In such a clinical examination, plural instruments are used for the centrifugal separation and signal-measurement. Thus, a trained resident operator is indispensable.

Jpn. Pat. Appln. KOKOKU Publication No. 55-36937 discloses a rotary cuvette type rotor for a photometer that can be operated without the aid of gravity. This photometer rotor has a structure for attaining operations from centrifugal separation to reagent-reaction by means of a single machine. The photometer rotor is equipped with a laminated disk having two transparent disks (front and rear surface side transparent disks) and an opaque disk sandwiched between these disks.

Near the outer circumference of the laminated disk, sample-analyzing cuvettes in the form of ringed lines are made open in the opaque disk. The upper and lower surfaces of cuvettes are sealed up with the transparent disks at the front and rear sides. At the center of the front surface of the laminated disk is located a sample-distributing chamber sectioned by a groove made in the front surface of the opaque disk and the front surface side transparent disk. A sample injecting port is made open at the center of the front surface side transparent disk, and connected to the sample-distributing chamber. The sample-distributing chamber is connected to the individual cuvettes through bent channels sectioned by a narrow groove made in the front surface of the opaque disk and the front surface side transparent disk.

At the center of the rear surface of the laminated disk is located a reagent-distributing chamber sectioned by a groove made in the rear surface of the opaque disk and the rear surface side transparent disk. A reagent injecting port is made open at the center of the rear surface side transparent disk, and connected to the reagent-distributing chamber. The reagent-distributing chamber is connected to the individual cuvettes through channels that are sectioned by a narrow groove made in the rear surface of the opaque disk and the rear surface side transparent disk, and are linearly extended in the radial direction.

In a clinical examination of a specimen (for example, a blood) by means of the rotor, for a photometer, having this structure, a measuring operator puts a fixed amount of a reagent from the reagent injecting port to the reagent-distributing chamber with a pipette or some other means, and further puts the blood from the sample injecting port to the sample-distributing chamber. Thereafter, the rotor is rotated to send the reagent in the reagent-distributing chamber into the sample-analyzing cuvettes, and blood plasma separated by centrifugation from the blood in the sample-distributing chamber is sent into the sample-analyzing cuvettes, thereby causing the blood plasma and the reagent to react with each other in the cuvettes.

### Citation List

### Patent Document

Patent Document 1: Jpn. Pat. Appln. KOKOKU Publication No. 55-36937

### Disclosure of Invention

### Problem to Be Solved

However, in a clinical examination of a specimen, such as blood, with the rotary cuvette type rotor, for a photometer, described in Jpn. Pat. Appln. KOKOKU Publication No. 55-36937, the reagent-distributing chamber is connected to the sample-analyzing cuvettes through the channels extended linearly in the radial direction; it is therefore difficult for the reagent supplied into the distributing chamber by centrifugal force when the rotor is rotated to be distributed evenly into the individual cuvettes with a desired precision. As a result, the reagent amount which is to react with blood plasma varies between the individual cuvettes. Thus, the analysis precision of a blood component such as glucose in the blood plasma falls.

Jpn. Pat. Appln. KOKOKU Publication No. 55-36937 also states that in order to cause different reagents between the individual cuvettes to react, in the cuvettes, with blood plasma separated by centrifugation, the different reagents are beforehand put into the individual sample-analyzing cuvettes and then the reagents are freeze-dried. The document states that the thus-freeze-dried reagents are made into individual solutions by distributing water or buffer liquid supplied into the reagent-distributing chamber into the individual cuvettes in a manner equivalent to the above-mentioned manner of distributing the reagent.

However, when the water or buffer liquid is distributed from the reagent-distributing chamber into the individual cuvettes, it is difficult, in the same manner as when the reagent is distributed, for the water or buffer liquid to be evenly distributed into the individual cuvettes with a desired precision. As a result, even when a desired amount of the reagent is beforehand put into each of the cuvettes and the reagent is freeze-dried, the distribution amount of the water or buffer liquid, for making the freeze-dried reagent into the solutions, varies so that the reagent concentrations between the individual cuvettes are different from each other. Accordingly, the analysis precision of a blood component such as glucose in blood plasma falls because of the variation in the reagent concentration between the individual cuvettes.

An object of the present invention is to provide a clinical examination disk capable of causing a specimen, such as blood plasma separated by centrifugation from a blood, to react, in a reaction tank, with a reagent held therein with a desired precision.

Another object of the invention is to provide a disk pack capable of holding stably a gel-form reagent held beforehand in a reaction tank of a clinical examination disk.

Still another object of the invention is to provide a clinical examination device capable of maintaining the air-tightness of the inside of a space (chamber) wherein the above-mentioned clinical examination disk is arranged, and further attaining the control of the temperature of the device with a high precision.

### Means for Solving the Problem

According to a first aspect of the invention, there is provided a clinical examination disk, comprising a disk body in a disk form, and at least one cell formed in the disk body,
wherein the cell comprises:
a specimen reservoir formed in the disk body and having a specimen injecting port;
a reaction tank formed in the disk body so as to position nearer to the outer circumference of the body than the specimen reservoir, and holding a reagent which is reactive with the specimen and is beforehand gelatinized;
a first narrow tube formed in the disk body, and communicating to the specimen reservoir and the reaction tank; and
a second narrow tube formed in the disk body, and having one end connected to the reaction tank and the other end made open.

According to a second aspect of the invention, there is provided a disk pack, comprising a moisture-proof bag, the clinical examination disk that is air-tightly held in the bag, and a moisture-adjusting agent held, together with the clinical examination disk, in the bag.

According to a third aspect of the invention, there is provided a clinical examination device, comprising:
an outer packaging member;
a case arranged in the outer packaging member and having a chamber inside the case;
a linearly-acting unit comprising a base plate arranged in the chamber of the case to be vertically movable;
a passage hole made open a portion which is extended from an upside portion of a side wall of the case to a side wall of the outer packaging member;
a tray which is capable of putting into the chamber through the passage hole and taking out the chamber through the passage hole, and having a circular concave onto which a clinical examination disk as described above is placed; and
a rotating unit fixed to the linearly-acting unit to cause the disk to be rotated,
wherein the tray comprises a ringed hole made open in a ringed region around the outer circumference of the circular concave, and a ringed packing fixed to the ringed hole to be vertically movable,
the base plate comprises, on an upper surface thereof, a pushing-up member to push up the ringed packing, and
a thin-film heater unit is formed on at least one of the inner surface of an upper wall of the case and a lower surface of the tray.

### Advantage of the Invention

According to the invention, it is possible to provide a clinical examination disk capable of analyzing a specimen, for example, a blood component, such as blood glucose, with a high precision.

According to the invention, it is possible to provide a disk pack capable of holding stably a gel-form reagent held beforehand in a reaction tank of a clinical examination disk.

According to the invention, it is possible to provide a clinical examination device that enables precise control over the temperature of reaction between a specimen (for example, blood plasma separated by centrifugation from a blood) and a reagent in a cell of the above-mentioned clinical examination disk which is rotated at a high speed, as well as even liquification of a gelatinized reagent in a reservoir formed in the cell of the disk, before the reaction, thus making it possible to attain a blood examination high in reproducibility.

### Brief Description of Drawings

FIG. 1 is a plan view illustrating a clinical examination disk according to an embodiment;
FIG. 2 is a view illustrating a cell in FIG. 1 in an enlarged state;
FIG. 3 is a perspective view illustrating an examination device using fluorescence;
FIG. 4 is a plan view showing a positional relationship between individual photo-interrupters and a point where excited light is radiated when the disk in FIG. 1 is set to the examination device;
FIG. 5 is an enlarged plan view illustrating a cell of a different clinical examination disk according to the embodiment;
FIG. 6 is an enlarged plan view illustrating a cell of a different clinical examination disk according to the embodiment;
FIG. 7 is an enlarged plan view illustrating a cell of a different clinical examination disk according to the embodiment;
FIG. 8 is an enlarged plan view illustrating a cell of a different clinical examination disk according to the embodiment;
FIG. 9 is an enlarged plan view illustrating a cell of a different clinical examination disk according to the embodiment;
FIG. 10 is an enlarged plan view illustrating a cell of a different clinical examination disk according to the embodiment;
FIG. 11 is a view illustrating a cell of a different clinical examination disk according to the embodiment in an enlarged state;
FIG. 12 is a view showing the effect (of the cell) in FIG. 11;
FIG. 13 is a sectional view of a cell of a different clinical examination disk according to the embodiment;
FIG. 14 is a rear view of a cell of a clinical examination disk according to the embodiment;
FIG. 15 is a rear view of a cell of a different clinical examination disk according to the embodiment;
FIG. 16 is an exploded perspective view of a different clinical examination disk according to the embodiment;
FIG. 17 is a view illustrating the cell in FIG. 16 in an enlarged state;
FIG. 18 is an exploded perspective view illustrating a disk package according to the embodiment;
FIG. 19 is a schematic perspective view of a clinical examination device according to the embodiment;
FIG. 20 is a sectional view of the clinical examination device in FIG. 19;
FIG. 21 is a sectional view of a main portion of the clinical examination device in FIG. 19;
FIG. 22 is a sectional view referred to in order to describe the operation of the clinical examination device in FIG. 19; and
FIG. 23 is a sectional view referred to in order to describe the operation of the clinical examination device in FIG. 19.

### Mode for Carrying Out the Invention

With reference to some of the drawings, a clinical examination disk and a disk pack according to an embodiment of the invention will be described in detail hereinafter.

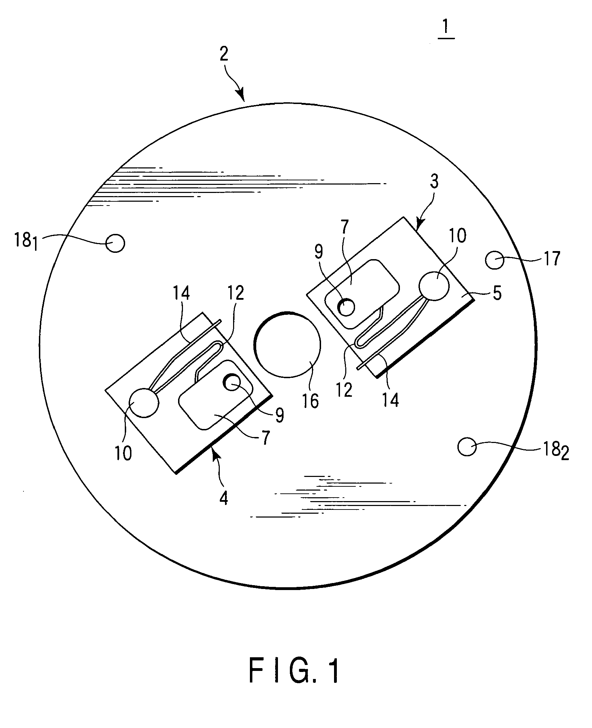

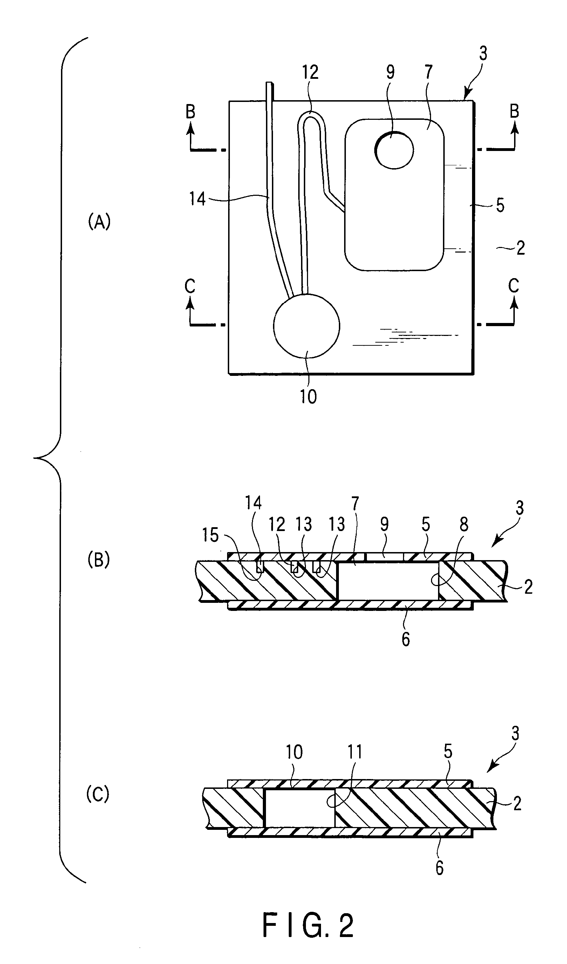

FIG. 1 is a plan view illustrating the clinical examination disk according to the embodiment. FIG. 2A is an enlarged plan view illustrating one of the cells in FIG. 1, FIG. 2B is a sectional view thereof taken along line B-B in FIG. 2A, and FIG. 2C is a sectional view thereof taken along line C-C in FIG. 2A.

The clinical examination disk 1 includes a disk body 2 in a disk form. In the disk body 2, for example, two cells (a specimen examining cell 3 and a calibrating cell 4) are formed centrosymmetrically with respect to the center thereof. As illustrated in FIG. 2B and 2C, the specimen examining cell 3 is composed of the disk body 2, and rectangular, front and rear side window plates 5 and 6 which have the same size and are adhered to the front and rear surfaces of the disk body 2, respectively, so as to be opposed to each other. The window plates 5 and 6 are each made of a transparent or semitransparent synthetic resin. The process for adhering (i.e., attaching and bonding) the window plates 5 and 6 may be, for example, bonding with a UV adhesive, bonding with a solvent, melt-bonding by heat (with a heater), ultrasonic melt-bonding, or laser melt-bonding. The adhering process is in particular suitably laser melt-bonding. A rectangular specimen reservoir 7 is formed by sealing up a rectangular through hole 8 made open in the disk body 2 with the front and rear side window plates 5 and 6. A specimen injecting port 9 is made open in a portion of the front side window plate 5 that corresponds to the rectangular through hole 8. A circular reaction tank 10 is formed by sealing up, with the front and rear side window plates 4 and 5, a circular through hole 11 made open in the disk body 2 and positioned nearer to the outer circumference of the disk body 2 than a position of the specimen reservoir 7. The reaction tank 10 holds therein a gelatinized reagent beforehand.

When a specimen (for example, a blood) is used and the blood is centrifuged into a supernatant (blood plasma) and blood cells in the specimen reservoir 7, one end of a first narrow tube 12 is connected to a region of the specimen reservoir 7 where the supernatant is retained, and the other end thereof is connected to the reaction tank 10. In other words, the first narrow tube 12 functions as a channel for introducing the blood plasma in the specimen reservoir 7 into the reaction tank 10. This first narrow tube 12 is formed by covering, with the front surface side window plate 5, a groove 13 worked in the front surface of the disk body 2 and positioned between the rectangular through hole 8 and the circular through hole 11. With regard to a second narrow tube 14, one end thereof is connected to the reaction tank 10 while the other end thereof is made open. In other words, when a separated material (for example, blood plasma in the blood) from the specimen centrifuged in the specimen reservoir 7 is introduced into the reaction tank 10, the second narrow tube 14 functions as a channel for causing the air in the reaction tank 10 to escape to the outside. This second narrow tube 14 is formed by covering, with the front surface side window plate 4, most of a groove 15 made in the front surface of the disk body 2 so as to position from the circular through hole 11 toward the outside of the front surface side window plate 5.

In the same manner as the specimen examining cell 3, the calibrating cell 4 includes a reservoir 7 having a specimen injecting port 9, a reaction tank 10 wherein a gelatinized reagent is beforehand held, and first and second narrow tubes 12 and 14. A gelatinized calibrating standard sample is previously held in the reservoir 7 of the calibrating cell 4. When the temperature of the sample is returned to room temperature, the sample is liquefied. The calibrating standard sample has, for example, a composition containing a buffer liquid for making a pH constant, a measurement target substance, a fixed amount of which is dissolved in this buffer liquid, and rabbit blood plasma added to the buffer liquid in order to cause the contact angle to approach the contact angle of the blood plasma in the blood, which is the above-mentioned specimen.

A supporting hole 16 is made open at the center of the disk body 2. A rotating axis of a disk rotating motor is inserted into the hole. A rotation-number-detecting hole 17 is made open, for example, in the vicinity of the outer circumferential edge of the disk body 2 that is in the form on a straight line with which the center of the supporting hole 16 is jointed to the center of the reaction tank 10 of the specimen examining cell 3. Timing detecting holes 18₁ and 18₂, the number of which is two corresponding to the number of the cells 3 and 4, are made open so as to position at the periphery of the disk body 2 but nearer to the disk center than a position of the rotation-number-detecting hole 17, and so as to have an angle of 180° therebetween. The timing detecting holes 18₁ and 18₂ are each positioned to make a desired angle to the rotation-number-detecting hole 17, and are further positioned asymmetrically with respect to the hole 17. The positions where the timing detecting holes 18₁ and 18₂ are made open in the disk body 2 are decided in accordance with a positional relationship between the following points when the disk is set to a fluorescence-used examination device that will be described later: a radiation point where from a light emitting element of its timing-detecting photo-interrupter, light is radiated to the disk; and an excited-light radiation point where from its excited-light-radiating unit, excited light is radiated to the disk. One of the timing detecting holes, that is, the hole 18₁ is used to decide the timing of excited light radiation to the reaction tank 10 of the specimen examining cell 3. The other timing detecting holes 18₂ is used to decide the timing of excited light radiation to the reaction tank 10 of the calibrating cell 4.

The disk body 2 has, for example, the following dimensions: a thickness of 1 mm, and a diameter of 120 mm. The disk body 2 is formed by injection-molding a synthetic resin such as black polyethylene, polypropylene, polystyrene, ABS, acrylic resin, polycarbonate, cycloolefin, or POM.

Examples of the transparent or semitransparent synthetic resin used in the front and rear surface side window plates 5 and 6 include polyethylene terephthalate (PET), polyethylene, polypropylene, polystyrene, ABC, polyacrylic acid, polycarbonate, and polycycloolefin.

Each of the gelatinized reagents can be prepared by pre-holding a solution (sol), which is prepared by adding a gelatinizer to a reagent, into one of the reaction tanks 10 beforehand, and then refrigerating and storing the clinical examination disk 1 to gelatinize the solution. When the gelatinized reagent is refrigerated at, for example, 10°C or lower, the reagent is gelatinized so that the fluidity thereof is lost. Thus, the gelatinized reagent does not leak out from the reaction tank 10 by vibration when the disk 1 is carried. However, when the temperature of the gelatinized reagent in the reaction tank 10 turns to 20°C or higher, the reagent is liquefied so that the fluidity thereof is expressed. The liquefied reagent can be mixed with a specimen and caused to react therewith in the same manner as an ordinary liquid reagent.

Examples of the gelatinizer include gelatin, sodium alginate, carrageenan, and pectin. These gelatinizers may be used single or in the form of a mixture of two or more thereof. It is preferred to add the gelatinizer in such an amount that the gelatinized reagent can be liquefied at a temperature of 20°C or higher. In the case of gelatin, the addition amount thereof is preferably about 1% by weight.

The following will describe the effect of the clinical examination disk according to the embodiment with reference to some of the drawings.

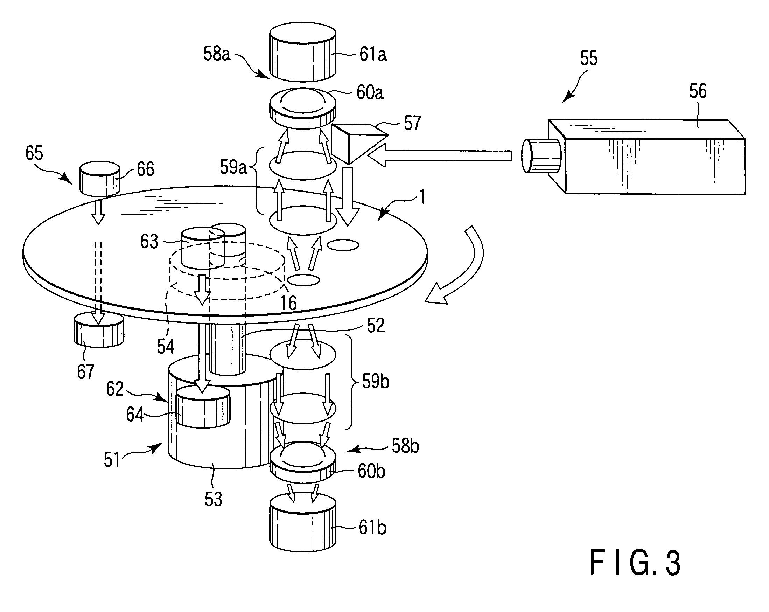

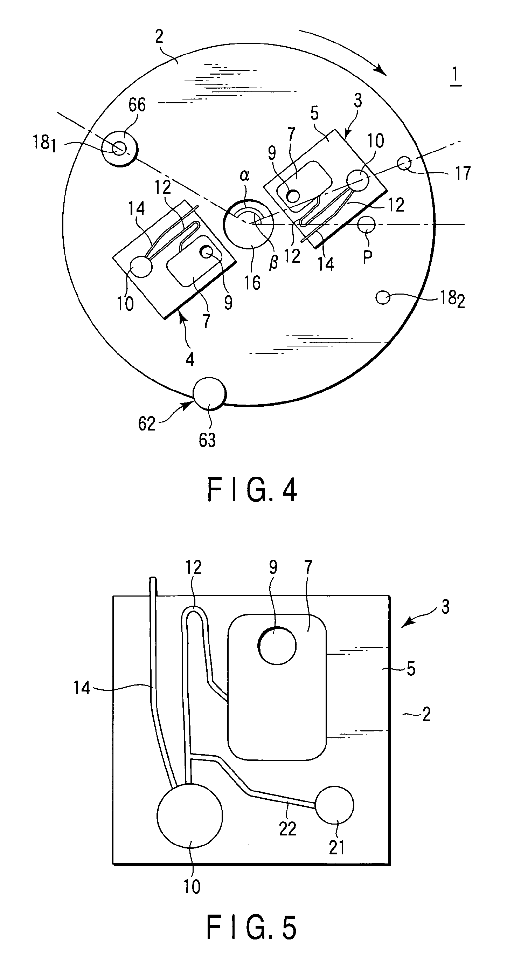

FIG. 3 is a perspective view illustrating an examination device using fluorescence, and FIG. 4 is a plan view showing a positional relationship between a rotation-number-detecting photo-interrupter, a timing-detecting photo-interrupter, and a point where excited light is radiated from an excited-light-radiating unit when the disk in FIG. 1 is set to the examination device.

The examination device includes a rotationally driving mechanism 51 which supports the disk 1 to be attachable to the mechanism and detachable therefrom, and causes the disk 1 to be rotated. The rotationally driving mechanism 51 is composed of a disk driving motor 53 having a driving axis 52 which is extended vertically and is rotatable in a clockwise direction, and a supporting disk 54 through which this driving axis 52 is penetrated and inserted and to which the axis 52 is fixed. In the rotatably driving mechanism 51 having this structure, the disk 1 is put onto the supporting disk 54 in the state that a taking-out hole 16 therein is fitted onto the driving axis 52. A clamper (not illustrated) is engaged with the driving axis 52 projected from the disk 1, and the disk is sandwiched between the supporting disk 54 and the clamper, so as to be fixed.

An excited-light-radiating unit 55 is arranged in the vicinity of the supporting disk 54 of the rotatably driving mechanism 51. The excited-light-radiating unit 55 is composed of an exciting light source (for example, an N₂ laser instrument) 56, and an optical element (for example, a mirror) 57 for introducing laser light emitted out from this N₂ laser instrument 56 to reactants in the reaction tanks 10 in the cells 3 and 4 of the disk 1.

A pair of fluorescence receiving units 58a and 58b are arranged around the supporting disk 54 to make a desired interval angle to the excited-light-radiating unit 55 in the clockwise direction from the unit 55, respectively. The fluorescence receiving units 58a and 58b receive fluorescence emitted when excited light is radiated to the reactant in any one of the reaction tanks 10 of the disk 1, respectively, and then output an electric signal in accordance with the received light amount. The paired fluorescence receiving units 58a and 58b are arranged in such a manner that when the disk 1 is set onto the supporting disk 54, the units 58a and 58b are positioned over and under the disk 1, respectively, so that the disk 1 is sandwiched therebetween. One of the fluorescence receiving units, that is, the unit 58a (at the upper side) is composed of an optical element (for example, a relay lens) 59a for receiving fluorescence emitted from a reactant in any one of the reaction tanks 10 of the disk 1, an interfering filter 60a, and a photoelectron multiplier tube 61a, these members 59a, 60a and 61a being arranged successively upward from the vicinity of the disk 1. The other of the fluorescence receiving units, that is, the unit 58b (at the lower side) is composed of an optical element (for example, a relay lens) 59b for receiving fluorescence emitted from a reactant in any one of the reaction tanks 10 of the disk 1, an interfering filter 60b, and a photoelectron multiplier tube 61b, these members 59b, 60b and 61b being arranged successively downward from the vicinity of the disk 1.

A disk-rotation-number-detecting photo-interrupter 62 counts the rotation number of the disk 1. The photo-interrupter 62 is arranged around the supporting disk 54 to make a desired interval angle to each of the fluorescence receiving unit 58a and 58b in the clockwise direction from the unit. The photo-interrupter 62 is composed of a light emitting element 63 arranged over the disk 1, and a light receiving element 64 arranged under the disk 1. In this photo-interrupter 62, light from the light emitting element 63 is radiated downward. When the rotation-number-detecting hole 17 in the disk 1 which is being rotated is passed just under the light emitting element 63 and then the light from the element 63 is radiated through the rotation-number-detecting hole 17 into the light receiving element 64, a pulse signal is output from the light receiving element 64.

The timing-detecting photo-interrupter 65 controls the timing of radiating excited light from the excited-light-radiating unit 55 into the reaction tank 10 of any one of the cells 3 and 4 of the disk 1. The photo-interrupter 65 is arranged around the supporting disk 54 to make a desired interval angle to the disk-rotation-number-detecting photo-interrupter 62 in the clockwise direction from the photo-interrupter 62. The photo-interrupter 65 is composed of a light emitting element 66 arranged over the disk 1 and a light receiving element 67 arranged under the disk 1. In this photo-interrupter 65, light from the light emitting element 66 is radiated downward. When the timing-detecting hole 18₁ (or 18₂) in the disk 1 which is being rotated is passed just under the light emitting element 66 and then the light from the element 66 is radiated through the timing-detecting hole 18₁ (or 18₂) into the light receiving element 67, a pulse signal is output from the light receiving element 65. When this pulse signal is output into the excited-light-radiating unit 55, laser light (excited light) is emitted from the N₂ laser instrument 56 thereof. The emitted excited light is radiated through the optical element 57 into the reaction tank 10 of the specimen examining cell 3 (or the calibrating cell 4) of the disk 1. When the pulse signal is output into the excited-light-radiating unit 55, a time lag is applied to the signal, thereby performing timing-adjustment.

In a clinical examination (for example, a blood examination), the temperature of the refrigerated and stored disk 1 is first returned to room temperature. At this time, the gelatinized reagents held beforehand in the respective reaction tanks 10 of the specimen examining cell 3 and the calibrating cell 4, and the gelatinized calibrating standard sample held beforehand in the reservoir 7 of the calibrating cell 4 are each liquefied. The used reagents are two antibodies each labeled with a colorant. A desired amount of a specimen (for example, a blood) is injected through the specimen injecting port 9 into the specimen reservoir 7 of the specimen examining cell 3.

Next, the supporting hole 16 at the center of the disk 1 is fitted onto the driving axis 52 of the rotatably driving mechanism 51, as described above. At this time, the disk 1 is put on the supporting disk 54 and is further caught together with the supporting disk 54 with the clamper (not illustrated), so as to be fixed. When the motor 53 of the rotatably driving mechanism 51 is driven to rotate the driving axis 52 thereof in a clockwise direction, the supporting disk 54 and the disk 1 fixed with the clamper are rotated at a speed of, for example, 10000 rpm. At this time, the blood injected in the specimen reservoir 7 of the specimen examining cell 3 is centrifuged so that the blood is separated into blood cells at the disk 1 outer circumferential side of the specimen reservoir 7 and a supernatant (blood plasma) at the disk 1 central side of the specimen reservoir 7. In the centrifugation, a centrifugal force larger than the capillary force of the first narrow tube 12 connected to the specimen reservoir 7 is applied to the blood in the specimen reservoir 7. For this reason, it does not occur that the blood which is being centrifuged in the reservoir 7 is sent through the first narrow tube 12 into the reaction tank 10. When a brake is applied for stopping the driving of the motor 53 to lower the rotation number of the disk 1 gradually, the capillary force of the first narrow tube 12 exceeds the centrifugal force. Thus, by the capillary force, the blood plasma separated by the centrifugation in the reservoir 7 is passed from a middle position of the reservoir 7 in the longitudinal direction thereof into the reaction tank 10 through the first narrow tube 12 positioned at the periphery of the disk 1. At the same time, the air in the reaction tank 10 is discharged through the second narrow tube 14 to the outside of the cell 3. Since inertia acts in addition to the capillary force based on the braking effect, the reagent liquefied in the reaction tank 10, and the blood plasma are mixed with each other. By making the reaction tank 10, in particular, into a column form, the mixing of the liquefied reagent and the blood plasma is smoothly and evenly attained. When a reagent through which fluorescence is measured is used, the mixture is then incubated at a constant temperature of 40°C or higher for a given period.

In the calibrating cell 4 also, at the time of rotating the disk 1 at a high speed and then braking the disk 1, the calibrating standard sample liquefied is sent from the reservoir 7 through the first narrow tube 12 into the reaction tank 10 so as to be mixed with the liquefied reagent. Furthermore, the mixture is incubated for a given period.

After such incubation, the motor 53 of the rotatably driving mechanism 51 is again driven to rotate the disk 1 at a speed of, for example, 10000 rpm. At this time, in the disk-rotation-number-detecting photo-interrupter 62, light is radiated downward from the light emitting element 63, and the rotation-number-detecting hole 17 in the disk rotated in a clockwise direction is passed just below the light emitting element 63 so that the light is radiated through the rotation-number-detecting hole 17 into the light receiving element 64. In the light radiation into the light receiving element 64, the disk 1 is rotated at a speed of 10000 rpm; thus, a pulse signal is output from the light receiving element 64 once every six milliseconds. After the pulse signal is output from the light receiving element 64, the timing-detecting hole 18₂ in the disk 1 rotated in the clockwise direction is passed just below the light emitting element 66, which radiates light, and the light is radiated through the timing-detecting hole 18₂ into the light receiving element 67; at this time, in the timing-detecting photo-interrupter 65, a pulse signal is output from the light receiving element 65. Furthermore, when the timing-detecting hole 18₁ in the disk 1 rotated is passed just below the light emitting element 66 and light therefrom is radiated through the timing-detecting hole 18₁ into the light receiving element 67, a pulse signal is output from the light receiving element 65. The pulse signal output from the light receiving element 65, which follows the former penetration of the light through the timing-detecting hole 18₂, is ignored, and the pulse signal output from the light receiving element 65, which follows the latter penetration of the light through the timing-detecting hole 18₁, is input into the excited-light-radiating unit 55. At the time of the input of the pulse signal into the excited-light-radiating unit 55, laser light (excited light) emitted from the N₂ laser instrument 56 thereof is radiated through the optical element 57 into the reaction tank 10 of the specimen detecting cell 3 of the disk 1. The reactant therein is excited to emit fluorescence. At this time, the timing detecting hole 18₁ and the reaction tank 10 of the cell 3, which corresponds to this timing-detecting hole 18₁, have a positional relationship illustrated in FIG. 4. Specifically, the angle α made by two lines obtained at the time of jointing the timing-detecting hole 18₁, the center of the fitting hole 16 (driving axis 52) in the disk 1, and the center of the reaction tank 10 of the cell 3 is, for example, 6° smaller than the angle β made by two lines obtained at the time of jointing a position where light is radiated from the light emitting element 66 of the timing-detecting photo-interrupter 65 onto the disk 1, the center of the fitting hole 16 in the disk 1, and a point P where the excited light is radiated from the excited-light-radiating unit 55 to the disk 1. Thus, when the disk 1 is rotated by 6° from the time of the pulse-signal-output from the light receiving element 65, which follows the latter penetration of the light through the timing-detecting hole 18₁, the reaction tank 10 reaches the excited-light-radiated point P. The time required for the rotation of the disk 1 by 6° is 100 microseconds. When the motor 53 used for the rotation of the disk 1 is a brushless DC motor, wow flutter, which is unevenness in rotation, is generated at a ratio of about 1 to 4%. In this case, the rotation evenness while the motor is rotated for 100 microseconds corresponds to 1 microsecond. When this is converted into the shift distance of the reaction tank 10 of the cell 3, the distance is 40 µm. When the reaction tank 10 has a diameter of, for example, 8 mm, an accidental error of 40 µm is allowable. Accordingly, the effect of the wow flutter of the motor 53 is reduced so that after the output of the pulse signal from the light receiving element 65, which follows the penetration of the light through the timing-detecting hole 18₁, a remarkable improvement can be made in the precision of radiating the excited light from the excited-light-radiating unit 55 into the reaction tank 10 of the specimen detecting cell 3 of the disk 1.

After the radiation of the excited light into the reaction tank 10, and the fluorescence emission therefrom, intensities of two fluorescence species are each measured in a usual manner by means of the paired fluorescence receiving units 58a and 58b, which are positioned in the clockwise direction from the excited-light-radiated point P. Moreover, the following are attained: the radiation of excited light into the reaction tank 10 of the calibrating cell 4; the emission of two fluorescence species from the reactant in the reaction tank 10; and the measurement of the intensities of the two fluorescence species in the paired fluorescence receiving units 58a and 58b. When the fluorescence intensities measured in the reaction tank 10 of the specimen examining cell 3 are compared with standard values measured in the reaction tank 10 of the calibrating cell 4, for example, the concentration of the target substance in the blood plasma is examined.

The two timing-detecting holes 18₁ and 18₂ may be made open in the disk body 2 asymmetrically with respect to the disk-rotation-number-detecting hole 17. In other words, the positional relationship between the disk-rotation-number-detecting hole 17 and the timing-detecting holes 18₁ and 18₂ may be varied at the front and rear sides of the disk 1. According to this structure, from the time difference between the pulse-signal-output following the penetration of light through the disk-rotation-number-detecting hole 17 in the disk-rotation-number-detecting photo-interrupter 62 and that following the penetration of light through the timing-detecting hole 18₂ in the timing-detecting photo-interrupter 65, it can be detected whether the disk 1 is set to direct the front side thereof upward, or direct the rear side thereof upward. As a result, it can be decided whether the disk 1 is put onto the supporting disk 54 in a regular state or in an irregular state. In short, it is possible to prevent the disk from being set in error.

As described above, with regard to the clinical examination disk according to the embodiment, the gelatinized reagent is beforehand held in a fixed amount in the reaction tank of each of the cells; therefore, the reagent can be liquefied by exposing the disk to room temperature before an examination. In this state, a specimen (for example, a blood) is put into the specimen reservoir and then the above-mentioned examination device is used to rotate the disk to centrifuge the blood in the specimen reservoir, whereby at the time of sending the blood plasma separated by the centrifugation through the first narrow tube to the reaction tank, the blood plasma can be caused to react with the reagent, which has the above-mentioned target amount. Accordingly, by radiating excited light as described above into the reaction tank after the reaction and measuring fluorescence emitted from the reaction tank, the concentration of a specified component in the specimen (for example, the blood) can be measured with a high precision.

Each of the cells of the disk according to the embodiment has a structure wherein the second narrow tube is connected to the reaction tank, and an end of the second narrow tube is extended to the disk body outside the cell. In the cell having this structure, a specimen centrifuged in the specimen reservoir is sent through the first narrow tube into the reaction tank. At this time, the air in the reaction tank can be discharged through the second narrow tube to the outside of the cell. Thus, the centrifuged specimen can be smoothly and stably sent into the reaction tank.

With regard to the clinical examination disk according to the embodiment, the disk form thereof can be varied as described hereinafter.
(1) As illustrated in FIG. 5, the cell 3 may further include, in the disk body 2 thereof, a first overflow tank 21 formed so as to position nearer to the outer circumference of the disk body 2 than the specimen reservoir 7, and a third narrow tube 22 branched from the first narrow tube 12 and having a tip connected to the first overflow tank 21.

In the cell having this structure, a specimen (for example, a blood) is injected through the specimen injecting port 9 into the specimen reservoir 7. The blood plasma in the reservoir 7 after a centrifugation as described above is sent through the first narrow tube 12 into the reaction tank 10. In a case where the blood plasma is sent in an excessive amount into the reaction tank 10 at this time so that the reaction tank 10 overflows therewith, the blood plasma can be sent from the first narrow tube 12 through the branched third narrow tube 22 into the first overflow tank 21. As a result, the amount of the blood plasma into the reaction tank 10 can be made constant.

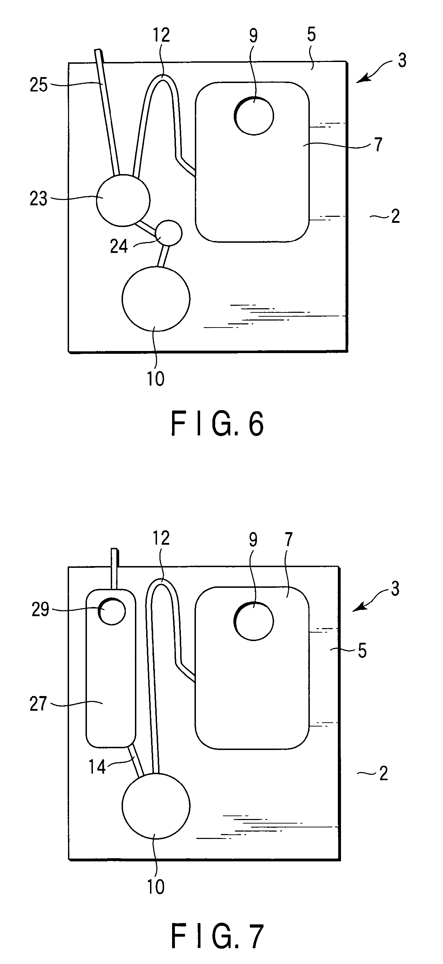

When a gelatinized reagent is beforehand held also in the first overflow tank 21, the overflow tank 21 can be used as a second reaction tank. Thus, another item of the specimen can be measured. In the case of making a measurement in a wide dynamic range, the measurement in the wide dynamic range can be attained by varying the diluting ratio (of a specimen).
(2) A cell 3 illustrated in FIG. 6 has a structure wherein a buffer tank 23 and an air trap 24 are further formed in a disk body 2 between a specimen reservoir 7 and a reaction tank 10, so as to arrang in this order from the vicinity of the specimen reservoir 7. A first narrow tube 12 is connected from the specimen reservoir 7 through the buffer tank 23 and the air trap 24 into the reaction tank 10. A fourth narrow tube 25 is formed in such a manner that one end thereof is connected to the buffer tank 23 in the disk body 2 and further the other end is made open. The buffer tank 23 and the air trap 24 can be formed by making a through hole in the disk body 2, respectively, and then sealing the through hole with window plates as described above, which are adhered onto the front and rear surfaces of the disk body 2.

In the cell 3 having this structure, a specimen (for example, a blood) is injected through a specimen injection port 9 into the specimen reservoir 7, and the disk is rotated at a high speed as described above. In this way, the blood is centrifuged into blood cells and blood plasma in the reservoir 7. When the rotated disk is braked to decrease the rotation number thereof, the blood plasma in the reservoir 7 is sent through the first narrow tube 12 into the buffer tank 23 by capillary force of the first narrow tube 12. At this time, the air in the buffer tank 23 is discharged through the fourth narrow tube 25 to the outside of the disk. When the rotation number of the disk 1 is again raised, the blood plasma in the buffer tank 23 is sent through the first narrow tube 12 and the air trap tank 24 by centrifugal force into the reaction tank 10. In other words, in accordance with the timing of the promotion of the rotation of the disk, the timing of sending the blood plasma into the reaction tank 10 and that of mixing the blood plasma with a reagent in the reaction tank 10 can be controlled. When the centrifugal force is lost, the air in the air trap tank 24 is inflated so as to produce a valve effect of blocking the liquid flow in the first narrow tube 12 with the air. It is therefore possible to prevent an excessive inflow of the blood plasma into the reaction tank 10, and the outflow of the reactant from the reaction tank 10. Such a cell structure is suitable for a reagent for which the reagent reaction time thereof is strictly managed. When the blood plasma is pushed from the buffer tank 23 into reaction tank 10, the air trap tank 24 acts to trap the air present in the reaction tank 10 to lower the resistance caused when the blood plasma is sent into the reaction tank 10. By arranging the buffer tank 23 inside the cell 3, the reactant in the reaction tank 10 can be kept in a wet state.
(3) In the embodiment, the reagent to which the fixed amount of the gelatinizer is added is directly held in the reaction tank 10 of the cell 3 in advance, and the disk is put into a refrigerator to gelatinize the reagent in the reaction tank 10, and stored. As illustrated in, for example, FIG. 7, a different configuration wherein a gelatinized reagent is held in a reaction tank in advance can be realized by forming a reagent reservoir 27 having an injecting port 26 in a disk body 2 region positioned at a second narrow tube 14. In a cell 3 having this reagent reservoir 27, a fixed amount of a reagent to which a gelatinizer is added is put from the injecting port 26 into the reagent reservoir 27. The disk is then rotated at a high speed so that the reagent in the reagent reservoir 27 is sent through the second narrow tube 14 into the reaction tank 10 by centrifugal force. Thereafter, the disk is put into a refrigerator to gelatinize the reagent in the reaction tank 10, and then the disk is stored. The reagent reservoir 27 is formed by making a through hole in the disk body 2 and then sealing up the through hole with window plates as described above, which are adhered onto the front and rear surfaces of the disk body 2.

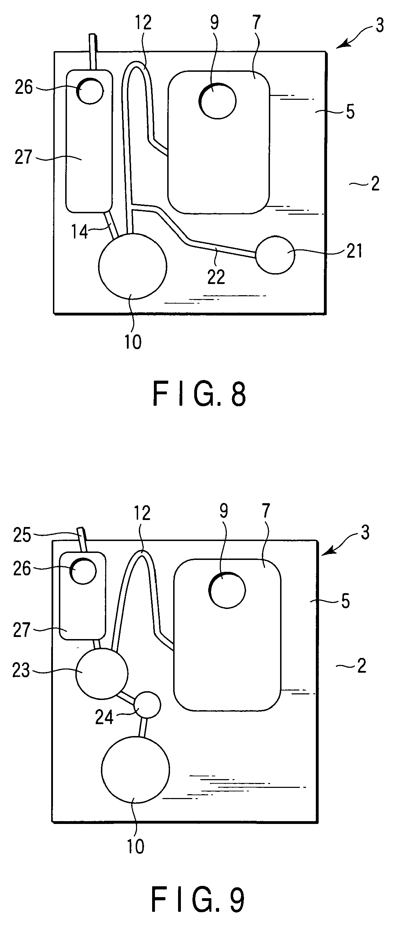

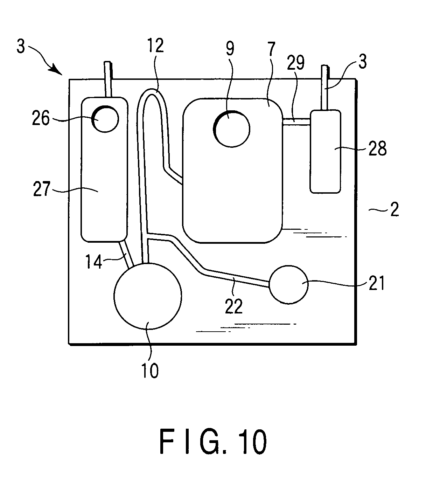

In the cell structure illustrated in FIG. 5, the reagent reservoir 27 having the above-mentioned function may be formed in the disk body 2 region positioned at the second narrow tube 14, as illustrated in FIG. 8. In the cell structure illustrated in FIG. 6, the reagent reservoir 27 having the above-mentioned function may be formed in the disk body 2 region positioned at the fourth narrow tube 25, as illustrated in FIG. 9.
(4) A cell 3 in FIG. 10 has a structure in which in the cell structure illustrated in FIG. 7, a second overflow tank 28 is further formed from the specimen reservoir 7 as a starting point so as to be adjacent to the specimen reservoir 7 along the circumferential direction of the disk body 2. The second overflow tank 28 is connected through a fifth narrow tube 29 to the specimen reservoir 7. The fifth narrow tube 29 is connected to a position of the specimen reservoir 7 which is nearer to the center of the disk body 2 than the connection position of the first narrow tube 12 connected to the specimen reservoir 7 to the center. With regard to a sixth narrow tube 30, one end thereof is extended to the second overflow tank 28, and the other end is extended to the disk body 2 outside the cell 3.

According to the cell 3 illustrated in FIG. 10, at the time of injecting a fixed amount of a specimen (for example, a blood) from the injecting port 9 into the specimen reservoir 7 and then rotating the disk at a high speed, the blood injected in the specimen reservoir 7 is centrifuged into a blood cell at the disk 1 outer circumferential side of the specimen reservoir 7 and a supernatant (blood plasma) at the disk 1 central side of the specimen reservoir 7. When a brake is applied for stopping the rotation of the disk 1 to decrease the rotation number of the disk gradually, the blood plasma separated by the centrifugation in the specimen reservoir 7 is sent from a middle position of the reservoir 7 in the longitudinal direction thereof into the reaction tank 10 through the first narrow tube 12 positioned at the outer circumferential side of the disk by capillary force thereof. Simultaneously, the blood cell separated by the centrifugation is sent from a middle position of the reservoir 7 in the longitudinal direction thereof into the second overflow tank 28 through the fifth narrow tube 29 positioned at the outer circumferential side of the disk by capillary force thereof. At this time, the air in the second overflow tank 28 is discharged through the sixth narrow tube 30 to the outside of the cell 3; thus, the blood cell separated by the centrifugation is smoothly sent into the second overflow tank 28.

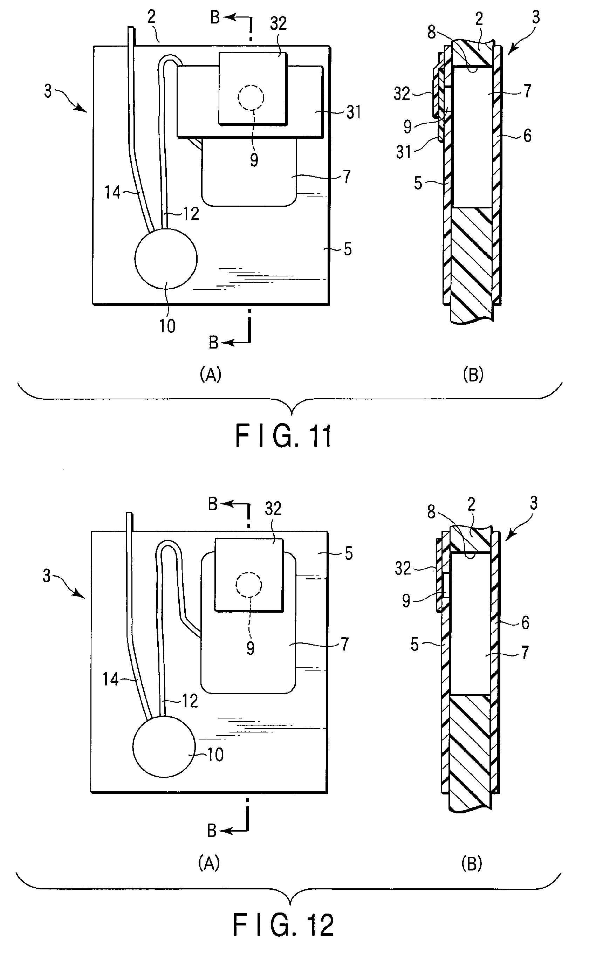

Accordingly, when the second overflow tank 28 is connected to the specimen reservoir 7 through the fifth narrow tube 29 with the predetermined positional relationship, the blood plasma separated in the specimen reservoir 7 can be sent into the reaction tank 10 in a target fixed amount.
(5) As illustrated in FIGS. 11A, 11B, 12A and 12B, a specimen examining cell 3 may be made into a structure wherein a specimen injecting port 9 in a specimen reservoir 7 of the cell 3 can be made open and closed. FIG. 11A is a plan view of the specimen examining cell 3, FIG. 11B is a sectional view thereof taken along line B-B in FIG. 11A, FIG. 12A is a plan view of the specimen examining cell 3, and FIG. 12B is a sectional view thereof taken along line B-B in FIG. 12A.

In a case where the specimen injecting port 9 in the clinical examination disk is kept open while the disk is rotated at a high speed, water in the cell 3 may evaporate. As illustrated in FIGS. 11A and 11B, a peeling mount 31 is arranged on a front surface side window plate 5 to cover a substantial half of a specimen reservoir 7 containing the specimen injecting port 9. An adhesive tape 32 is bonded onto an area extending from a region of the peeling mount 31 that is opposite to the specimen injecting port 9 to a front surface side window plate 5 of a region above the reservoir 7. In this structure, the peeling mount 31 can be inclined and moved (opened and closed) by aid of the adhesive tape 32. Thus, the specimen injecting port 9 can be covered with the peeling mount 31, and uncovered therewith. In order to inject a specimen into the specimen reservoir 7, the peeling mount 31 is moved to be inclined at 180° around the bonding point of the adhesive tape 29, thereby making the specimen injecting port 9 open. Subsequently to the injection of the specimen, as illustrated in FIGS. 12A and 12B, the peeling mount 31 is peeled from the adhesive tape 32 and then the adhesive tape 32 is bonded onto the front surface side window plate 5 in such a manner that the specimen injecting port 9 is closed with the adhesive tape 32. In this way, the specimen injecting port 9 is closed so as to make it possible to prevent the evaporation of water in the cell 3 composed of the reservoir 7, the reaction tank 9, and first and second narrow tubes 12 and 14. When a reagent for measuring fluorescence is used, the adhesive tape 32 is preferably an aluminum-evaporated tape or a tape having a black resin substrate.

This structure, wherein the specimen injecting port 9 in the specimen reservoir 7 can be made open and closed, may be applied to each of the cells illustrated in FIGS. 5, 6, 7, 8, 9 and 10.

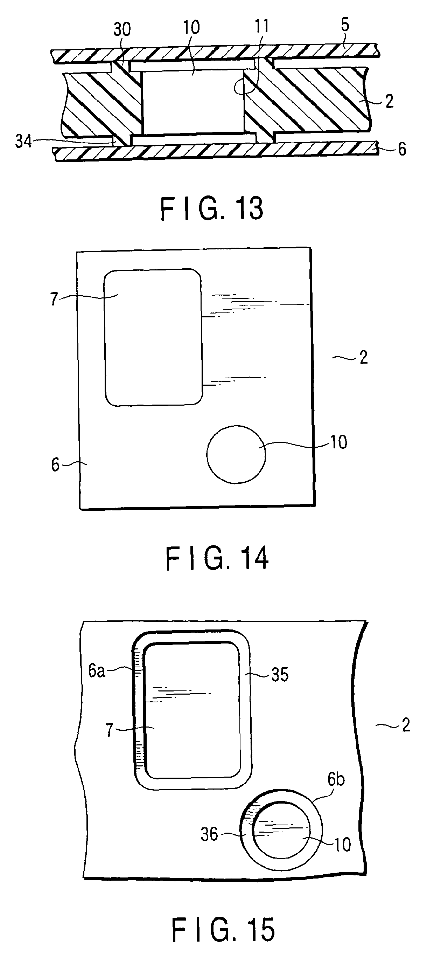

The injecting port 26 in the reagent reservoir 27 in each of FIGS. 7, 8, 9 and 10, which has been described in the items (3) and (4), may be made into a structure as illustrated in FIGS. 11A and 11B, wherein the port can be opened and closed.
(6) As illustrated in FIG. 13, circular ribs 33 and 34 are formed on the front and rear surface of a disk body 2, respectively, and around a circular through hole 10, so that the precision of the melt-bonding of front and rear side window plates 5 and 6 onto the disk body 2 is improved. In this way, a reaction tank having a fixed volume can be formed.

Specifically, in the clinical examination disk 1 according to the embodiment illustrated in FIGS. 1 and 2, a fixed amount of a gelatinized reagent is stored in the reaction tank 10, which is formed to have a fixed volume. By rotation at a high speed and centrifugation as described above, blood plasma is sent from the specimen reservoir 7 through the first narrow tube 12 into the reaction tank 10. The volume of the sent blood plasma is a value obtained by subtracting the volume of the reagent from the volume of the reaction tank 10. Fractional injections of the gelatinized reagent into the predetermined amount can be realized by a precise means such as a liquid-sending pump or pipette. Thus, for making the volume of the reaction tank 10 into a precise fixed value, important are the size of the circular through hole 11 in the disk body 2, which constitutes the reaction tank 10, and the attachment precision of the window plates 5 and 6 adhered onto the front and rear sides of the disk body 2. In the case of producing, for example, the reaction tank 10 having a volume of 50 µL, the window plates 5 and 6 are adhered onto the disk body 2 having the circular through hole 11 having a diameter of 8 mm, and having a thickness of 1 mm. Since the disk body 2 is formed by injection molding, variation between lots can be reduced. In the step of adhering the window plates 5 and 6 onto the front and rear surfaces of the disk body 2, respectively, an accidental error of 10 µm may be generated on any one of the two surface sides; in this case, the total thereof is 20 µm, so that a volume accidental error of 2% is generated. In short, the adhesion precision of the window plates 5 and 6 affects the volume accidental error of the reaction tank 10.

As illustrated in FIG. 13, in order to improve the adhesion precision of the window plates 5 and 6, circular ribs 33 and 34 are formed on the front and rear surfaces of the disk body 2, respectively, and around the circular through hole 10. When a laser melt-bonding device is used to melt-bond the window plates 5 and 6 onto the disk body 2, a melt-bonding receiving object wherein the window plates 5 and 6 are arranged onto the front and rear surfaces of the disk body 2, respectively, is set onto a table which can be vertically shifted and is arranged under a glass plate of the laser melt-bonding device. While the table is elevated to push the melt-bonding receiving object onto the lower surface of the glass plate, a laser ray is radiated onto the melt-bonding receiving object from above the glass plate. When the laser ray is radiated, the front surface side window plate 5 and the front surface of the disk body 2 are melted so that these members are melt-bonded. At this time, the laser ray is radiated onto the circular rib 33 on the front surface side and the pushing force of the melt-bonding receiving object onto the glass plate is controlled, thereby making it possible to control the amount of the circular rib 33 to be melted and squashed. After the front surface side window plate 5 and the front surface of the disk body 2 are melt-bonded to each other, the melt-bonding receiving object is overturned so that the circular rib 34 is positioned on the front surface side. Thereafter, according to the same procedure, the laser melt-bonding device is used to melt-bond the rear surface side window plate 6 onto the disk body 2 while the amount of the circular rib 34 to be melted and squashed is controlled. This melt-bonding operation makes it possible to control the distance between the front surface side window plate 5 and the rear surface side window plate 6 to a precise fixed value. As a result, it is possible to make consistent the volume of the reaction tank 10 formed by sealing up the circular through hole 11 in the disk body 2 with the front and rear surface side window plates 5 and 6.
(7) In the cell 3 formed in the disk body 2 in the embodiment, the rear surface side window plate 6 having the same dimension as the front surface side window plate is adhered onto the rear surface of the disk body 2 as illustrated in FIG. 14. However, the invention is not limited to this embodiment. The rear surface of the disk body 2 does not have any groove for forming a narrow tube; as illustrated in FIG. 15, therefore, a window plate divided into a window plate 6a for reservoir and a window plate 6b for reaction tank may be adhered onto the rear surface of the disk body 2. In FIG. 15, reference numbers 35 and 36 represent a region where the window plate 6a for reservoir and the disk body 2 are melt-bonded to each other, and a region where the window plate 6b for reaction tank and the disk body 2 are melt-bonded to each other, respectively. In a configuration wherein circular ribs are formed in the front and rear surfaces of a disk body and around a circular through hole and the circular ribs are squashed to melt-bond window plates onto the disk body as has been illustrated in FIG. 13, the division (of its window plate) into a window plate 6a for reservoir and a window plate 6b for reaction tank makes the volume precision of its reaction tank 10 still better since the control of the amount to be squashed becomes easy. With regard to the melt-bonding operation in this case, it is preferred to melt-bond the reaction tank window plate 6b initially onto the rear surface of the disk body 2, and subsequently melt-bond the reservoir window plate 6a onto the rear surface of the disk body 2.

In the clinical examination disk according to the embodiment, the disk body may be subjected, at the following regions thereof, to hydrophilicity-imparting treatment: the inner surface of the circular through hole for forming each of the reaction tanks; and the groove inner surfaces for making the narrow tubes. Examples of the hydrophilicity-imparting treatment include plasma treatment, excimer UV treatment, UV treatment, the painting of a hydrophilic polymer, the painting of a surfactant, and the immobilization of protein molecules.

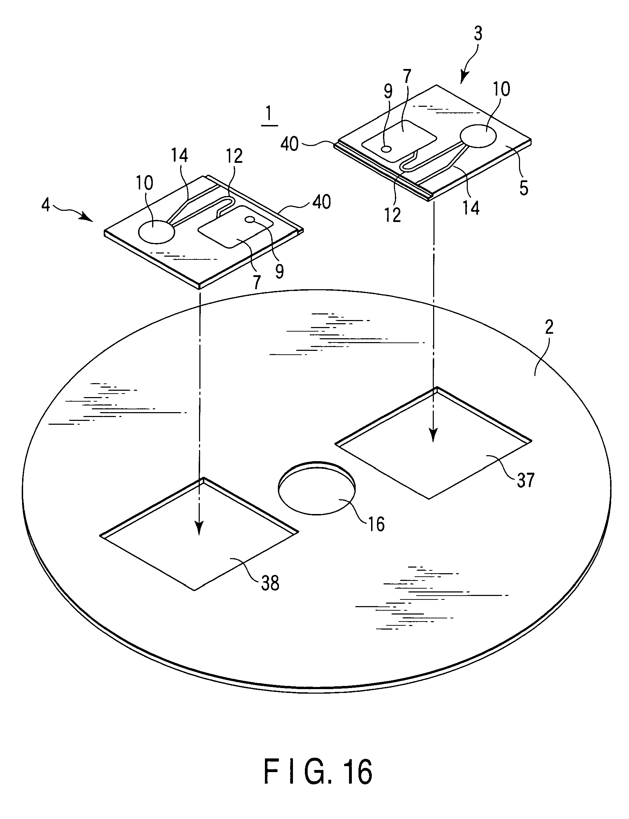

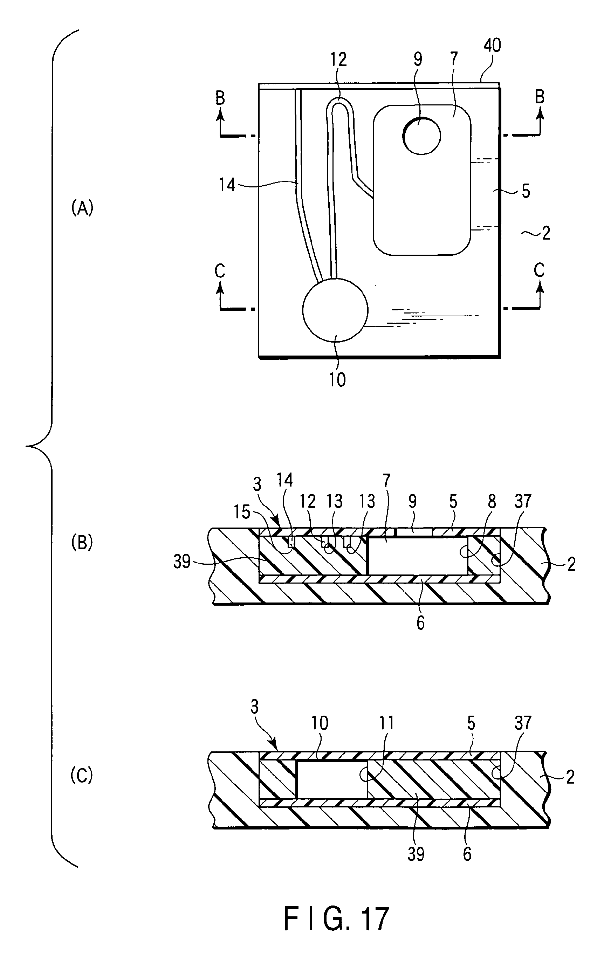

As illustrated in FIGS. 16, 17A, 17B and 17C, in another clinical examination disk according to the embodiment, for example, its two cells (a specimen examining cell 3 and a calibrating cell 4) may be arranged to be attached to a disk body 2 and detached therefrom. FIG. 16 is an exploded perspective view of this clinical examination disk of the embodiment. FIG. 17A is a plan view of a region of the specimen examining cell 3 arranged in the disk, FIG. 17B is a sectional view thereof taken along line B-B in FIG. 17A, and FIG. 17C is a sectional view thereof taken along line C-C in FIG. 17A.

The clinical examination disk 1 includes the disk body 2 in a disk form. In the disk body 2, for example, two rectangular grooves 37 and 38 for holding cells are formed centrosymmetrically with respect to a supporting hole 16. The disk body 2 has, for example, the following dimensions: a thickness of 1 mm, and a diameter of 120 mm. The disk body 2 is formed by injection-molding, for example, a black synthetic resin as described above. The disk body 2 may be made of colored glass. The specimen examining cell 3 and the calibrating cell 4 are arranged to be attachable to the rectangular grooves 37 and 38 in the disk body 2, respectively, and detachable therefrom, respectively.

As illustrated in FIGS. 17B and 17C, the specimen examining cell 3 is composed of a cell body 39, and rectangular front and rear surface side window plates 5 and 6 which have the same dimension and are adhered to the front and rear surfaces of the cell body 39, respectively, so as to be opposite to each other. The cell body 39 is formed by injection-molding, for example, a synthetic resin, such as black polyethylene, polypropylene, polystyrene, ABS, acrylic resin, polycarbonate, cycloolefin, or POM. A rectangular specimen reservoir 7 is formed by sealing up a rectangular through hole 8 made open in the cell body 39 with the front and rear surface side window plates 5 and 6. A specimen injecting port 9 is made open in a region of the front surface side window plate 5 which corresponds to the rectangular through hole 8. A circular reaction tank 10 is formed by sealing up, with the front and rear surface side window plates 4 and 5, a circular through hole 11 made open in the cell body 39 and positioned nearer to the outer circumference than a position of the specimen reservoir 7. In the reaction tank 10, a gelatinized reagent is beforehand held. A first narrow tube 12 is formed by covering, with the front surface side window plate 5, a groove 13 formed in the front surface of the cell body 39 and positioned between the rectangular through hole 8 and the circular through hole 11. A second narrow tube 14 is formed by covering, with the front surface side window plate 5, a groove 15 made in the cell body 39 so as to be extended from the circular through hole 11 toward the center of the disk body 2. An end of the groove 15 at the side thereof opposite to the circular through hole 11 is extended up to a position just before a step 40 of a cell body 39 side face positioned at the center (supporting hole 16) side of the disk body 2. In other words, in the state that the cell 3 is inserted and arranged in the rectangular groove 37 in the disk body 2, the end of the groove 15 is made open in the vertical wall of the step 40 to form an air escaping port in the reaction tank 10.

In the same manner as the specimen examining cell 3, the calibrating cell 4 is provided with a reservoir 7 composed of a cell body and front and rear surface side window plates, a reaction tank 10 wherein a gelatinized reagent is beforehand held, and first and second narrow tubes 12 and 14. In the reservoir 7 of the calibrating cell 4, a calibrating standard sample gelatinized is beforehand held. The sample is liquefied when the temperature of the disk 1 is returned to room temperature.

With regard to the clinical examination disk 1, wherein the specimen examining cell 3 and the calibrating cell 4 are arranged in the rectangular grooves 37 and 38 in the disk body 2, respectively, in this manner so as to be attachable thereto and detachable therefrom, after an examination of a specimen by use of the cells 3 and 4, only the cells 3 and 4 can be discarded and the disk body 2 can be reused. In other words, with regard to the clinical examination disk 1 illustrated in FIG. 1, wherein the cells 3 and 4 are attached to the disk body 2 so as to be integrated with the body 2, after an examination of a specimen by use of the cells 3 and 4, the whole of the disk body 2, that is, the disk 1 itself is discarded; thus, costs can be made lower according to the clinical examination disk 1 illustrated in FIGS. 16, 17A, 17B and 17C than according to the clinical examination disk 1 illustrated in FIG. 1.

Since the reagent gelatinized in advance is held in each of the reaction tanks 10, some container which contains this reagent is refrigerated and stored in, for example, a refrigerator. With regard to the clinical examination disk 1 illustrated in FIG. 1, the disk 1 itself is stored in the refrigerator. By contrast, with regard to the clinical examination disk 1 illustrated in FIGS. 16, 17A, 17B and 17C, only the cells 3 and 4 need to be stored in the refrigerator. Thus, it is possible to save space.

In the configuration wherein the cells can be arranged in the disk body to be attachable thereto and detachable therefrom, the cells are not limited to the structure illustrated in FIGS. 16, 17A, 17B and 17C. The cells may each be made into the structure illustrated in each of FIGS. 5, 6, 7, 8, 9 and 10.

In the clinical examination disk illustrated in FIGS. 16, 17A, 17B and 17C, the cell body may be subjected, at the following regions thereof, to hydrophilicity-imparting treatment: the inner surface of the circular through hole for forming each of the reaction tanks; and the groove inner surfaces for making the narrow tubes. Examples of the hydrophilicity-imparting treatment include plasma treatment, excimer UV treatment, UV treatment, the painting of a hydrophilic polymer, the painting of a surfactant, and the immobilization of protein molecules.

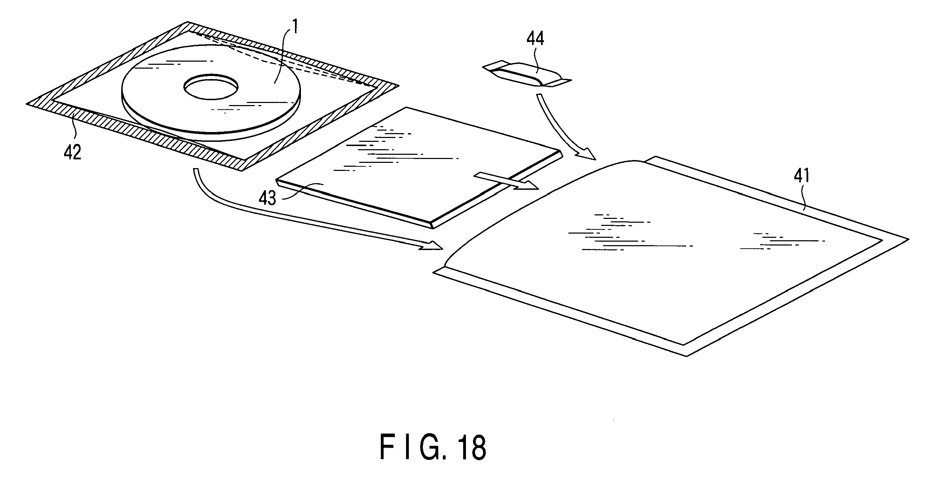

With reference to FIG. 18, the following will describe a disk package according to the embodiment.

The disk package according to the embodiment is composed of a bag 41, a small bag 42 held in this bag 41, a clinical examination disk 1 held in the small bag 42, a resin plate 43 which is held in the bag 41 and supports the small bag 42, and a humidity adjusting agent 44 held in the bag 41. The bag 41 is air-tightly sealed up by sealing an opening therein thermally after the small bag 42, the resin plate 43 and the humidity adjusting agent 44 are held therein.

The bag 41 is made of, for example, an aluminum-laminated film including thermally melt-bondable resin films laminated onto both sides of an aluminum foil piece, respectively, and a rigid resin film.

The small bag 42 is made of a resin film made of, for example, polyethylene terephthalate (PET), polyethylene, polypropylene or polystyrene.

The clinical examination disk 1 held in the small bag 42 may be the disk illustrated in FIG. 1, wherein the cells are integrated with the disk body, the disk having the cell structure in each of FIGS. 5, 6, 7, 8, 9 and 10, or the disk illustrated in FIGS. 16, 17A, 17B and 17C, wherein the cells can be arranged in the disk body to be attachable thereto and detachable therefrom.

The humidity adjusting agent may be, for example, sodium polyacrylate, or silica gel.

When the disk package having this structure is refrigerated and stored, the humidity in the bag 41 reaches about 100% by the cooling and the effect of the humidity adjusting agent 44. As a result, the gelatinized reagent held beforehand in each of the reaction tanks is prevented from being dried when the package is stored.

With regard to the embodiment, several cases where blood is used as a specimen have been described; however, the present invention may be used for all biological samples, such as bone marrow, blood serum, urine, and abdominal dropsy.

With reference to some of the drawings, the following will describe a clinical examination device according to the embodiment in detail.

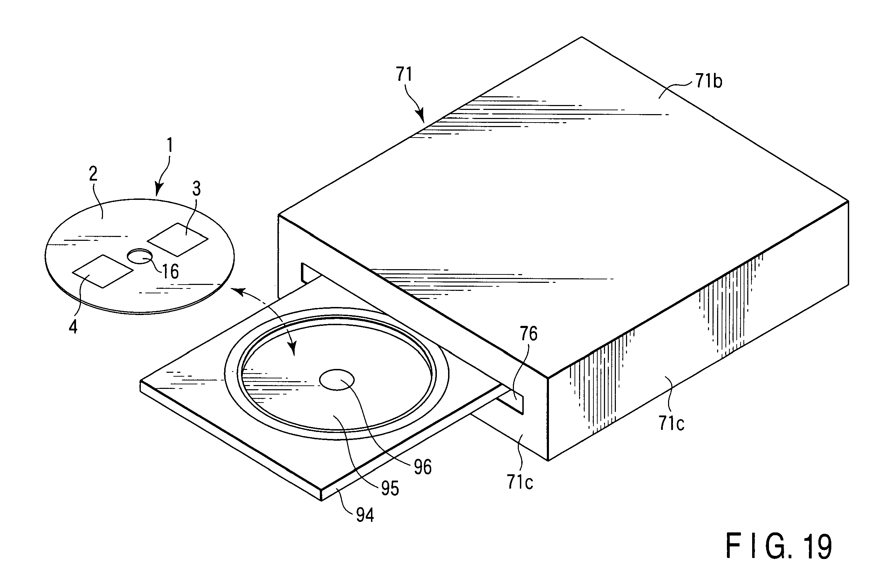

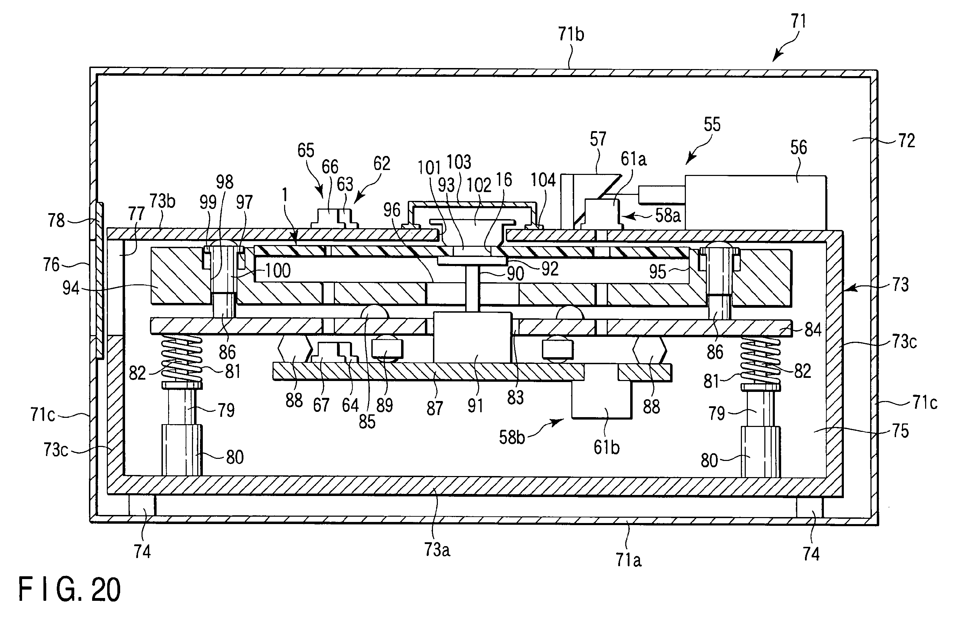

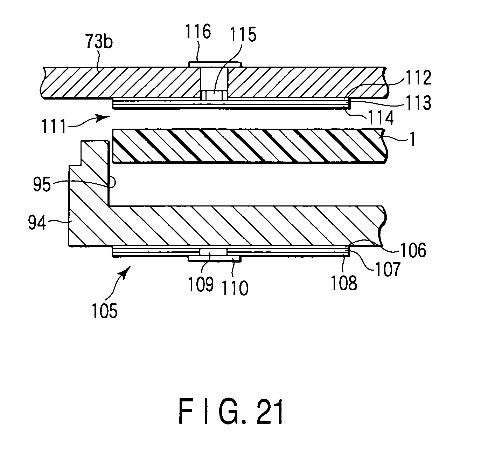

FIG. 19 is a schematic perspective view illustrating the clinical examination device according to the embodiment, FIG. 20 is a sectional view of the clinical examination device in FIG. 19, and FIG. 21 is a sectional view of a main portion of the clinical examination device in FIG. 19.

As illustrated in FIG. 19, the clinical examination device according to the embodiment has a structure similar to a front-loading type optical disk driver. An outer packaging member 71 is composed of a bottom plate 71a, a top plate 71b, and four side walls 71c, and has therein a rectangular space 72. A case 73 is arranged in the rectangular space 72 inside the outer packaging member 71, and fixed on legs 74 placed inside the outer packaging member 71 and on the bottom plate 71a. The case 73 is composed of a bottom plate 73a, an upper wall 73b, and four side walls 73c, and has therein a chamber 75. Passage holes 76 and 77 are made open in one of the side walls 71c of the outer packaging member 71 and one of the side walls 73c of the case 73, respectively, the two side walls being opposite to each other. A tray that can be reciprocated in the horizontal direction and will be described later is passed through the passage holes 76 and 77 to be put into the case and taken out therefrom. A shutter 78 is fitted to the inner surface of the side wall 71c of the outer packaging member 71 to be movable in the vertical direction, thereby opening and closing the passage holes 76 and 77.

Four cylinders 80 each having a piston 79 movable in the vertical direction are vertically set so as to position on the bottom plate 73a and at corners of the rectangle thereof inside the chamber 75. Four supporting axes 82 around each of which a coil spring 81 is wound are connected onto the pistons 7, respectively. A disk-form base plate 84 wherein a hole 83 is made open at the center thereof is fixed onto the tops of the four supporting axes 82. A first ringed packing 85 is fitted to the upper surface of the base plate 84 to surround the hole 83 therein. Pins 86, the number of which is, for example, 4, are located on the upper surface of the base plate 84 and outside the first ringed packing 85. The individual pins 86 each function as a pushing-up member for pushing up a ringed packing, this packing being inserted into the tray, which will be described later, so as to be movable in the vertical direction. A disk-form motor-placing plate 87 is arranged under the base plate 84 and connected to the lower surface of the base plate 84 through vibration-proof rubber members 88. A second ringed packing 89 is fixed between the base plate 84 and the motor-placing plate 87 so as to position inside the vibration-proof rubber members 88. The cylinders 80, the supporting axes 82, the base plates 84, the motor-placing plate 87 and other members constitute a linearly-acting unit.

In the linearly-acting unit having this structure, the pistons 79 of the cylinders 80 are raised. Thus, the supporting axes 82 connected to the individual pistons 79 are also raised. By the raise of these supporting axes 82, the base plate 84 fixed onto the individual supporting axes 82 is raised so that the motor-placing plate 87 connected to the base plate 84 is also raised. When the pistons 79 of the cylinders 80 are lowered, the motor-placing plate 87 together with the base plate 84 is lowered by the above-mentioned connecting structure.

A motor 91 having a rotating axis 90 extended upward is passed through the hole 83 in the base plate 84 so as to be fixed on the motor-placing plate 87 and at the center thereof. A disk-placing circular plate 92 is horizontally fixed onto the upper end of the rotating axis 90. A columnar body 93 fittable into the supporting hole 16 at the center of the clinical examination disk 1 is fixed onto the disk-placing circular plate 92 so as to be concentric with the rotating axis 90. The motor 91 having this rotating axis 90, the disk-placing circular plate 92, and the columnar body 93 constitute a rotating means.

A tray 94 is put through the passage holes 77 and 76 into a chamber 75 region between the base plate 84 and the upper wall 73b of the case 73, and taken out therefrom by means of a loading/unloading mechanism (not illustrated) arranged inside the chamber 75 of the case 73. The tray 94 is made of, for example, aluminum (or copper), which has a high thermal conductivity. The tray 94 is in a disk form, and therein a circular concave 95 is made concentrically therewith. A hole 96 having such a size that the disk-placing circular plate 92 connected to the rotating axis 90 can be inserted into the hole 96 is made open in the circular concave 95 and at the center thereof. In a tray 94 region outside the circular concave 95, a ringed hole 98 is made open which has at the upside thereof a step 97. A thick third ringed packing 100 having at the upside thereof a flange 99 is inserted into the ringed hole 98, and the flange 99 is engaged with the step 97 of the ringed hole 98, whereby the packing 100 is inserted therein to be vertically movable.

A hole 101 is made open in the upper wall 73b of the case 73 to be opposite to the disk-placing circular plate 92. A clamper 102 made of a magnet in an overturned-cap form is inserted into the hole 101 in the upper wall 73b from above. When the columnar body 93 on the disk-placing circular plate 92 is fitted into the supporting hole 16 in the disk 1, the magnetic clamper 102 is magnetically coupled with the columnar body 93 to be combined with the disk-placing circular plate 92, thereby sandwiching the disk 1 therebetween. An upper-region-closing cylindrical cover 103 is fixed onto the outer surface of the upper wall 73b with a fourth disk-form packing 104 interposed therebetween, so as to cover the clamper 102.

As illustrated in FIG. 21, a first thin-film heater unit 105 is arranged on a lower surface region of the tray 94 in which the circular concave 95 is positioned. In the state that the tray 94 is in a posture kept when the tray 94 is put into the case, the first thin-film heater unit 105 is set to a (tray) region except a circular region of the tray 94 lower surface that is extended toward the center from a base plate 84 region which contacts the first ringed packing 85. The first thin-film heater unit 105 is provided with a heat-radiating sheet 106 attached from the lower surface side of the tray 94, a film heater 107, and an aluminum plate 108 as a rear plate. The heat-radiating sheet 106 is made of, for example, an acrylic gel. The heat-radiating sheet 106 transmits heat from the film heater 107 evenly to the lower surface of the tray 94. A temperature sensor 109 is inserted into a hole made open to be extended over the aluminum plate 108 and the film heater 107, and is covered with a resin cover 110 adhered onto the aluminum plate 108. The temperature sensor 109 may be, for example, a thermocouple, or Pt100. The temperature of the film heater 107 is monitored with the temperature sensor 37. The monitor result is fed back to a temperature controller not illustrated so that the temperature of the film heater 107 is controlled.

A second thin-film heater unit 111 is set onto the inner surface of the upper wall 73b of the case 73 so as to be opposite to the first thin-film heater unit 105. The second thin-film heater unit 111 is provided with a film heater 112 adhered from the inner surface of the upper wall 73b, a heat-radiating sheet 113 and an aluminum plate 114. The heat-radiating sheet 113 is made of, for example, an acrylic gel. The heat-radiating sheet 113 transmits heat from the film heater 112 evenly to the aluminum plate 114 as an evenly heating plate. A temperature sensor 115 is inserted into a hole made open to be extended over the upper wall 73b and the film heater 112, and is covered with a resin cover 116 adhered onto the outer surface of the upper wall 73b. The temperature sensor 115 may be, for example, a thermocouple, or Pt100. The temperature of the film heater 112 is monitored with the temperature sensor 115. The monitor result is fed back to the temperature controller not illustrated so that the temperature of the film heater 112 is controlled.

In the same manner as the examination device in FIG. 3, the present device is provided with an excited light radiating unit 55 composed of an N₂ laser instrument 56 fixed onto the upper wall 73b of the case 73 and a mirror 57 for guiding a laser ray emitted from this N₂ laser instrument 56 to a reactant in each of the reaction tank of the cells in the disk 1. Out of the two fluorescence receiving units, one, 58a is composed of a relay lens (not illustrated), an interfering filter (not illustrated), a photoelectron multiplier tube 61a arranged on the upper wall 73b of the case 73, these members being arranged successively upward from the vicinity of the upper wall 73b. The other fluorescence receiving unit 58b (at the downside) is composed of a relay lens (not illustrated), an interfering filter (not illustrated), a photoelectron multiplier tube 61b fitted onto the motor-placing plate 87, these members being arranged successively downward. A disk-rotation-number-detecting photo-interrupter 62 is composed of a light emitting element 63 arranged on the upper wall 73b of the case 73, and a light receiving element 64 arranged on the motor-placing plate 87. A timing-detecting photo-interrupter 65 is composed of a light emitting element 66 arranged on the upper wall 73b of the case 73, and a light receiving element 67 arranged on the motor-placing plate 87.

The following will describe the effect of the clinical examination device with reference to FIGS. 19, 20, 22 and 23.

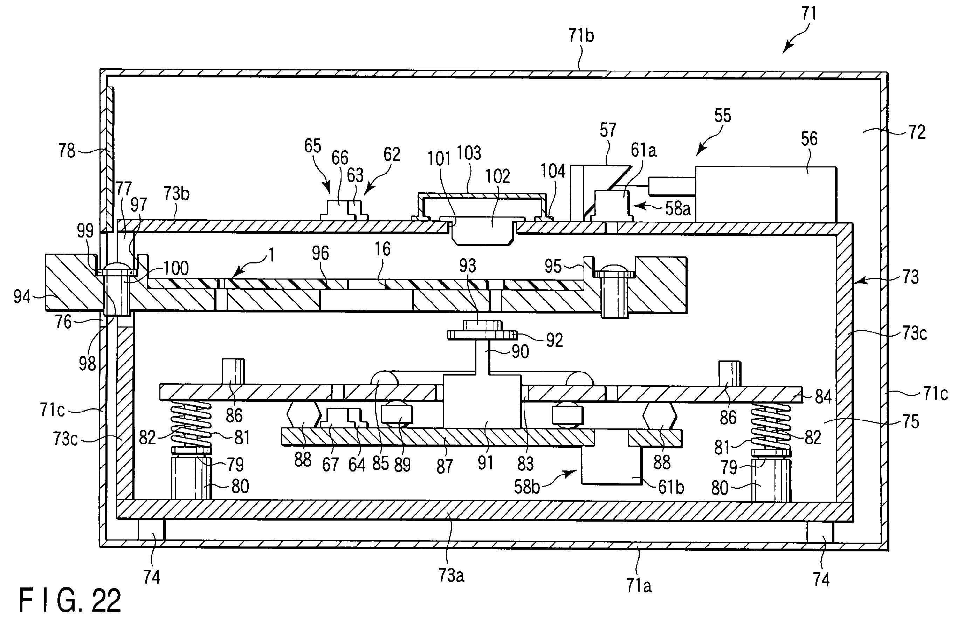

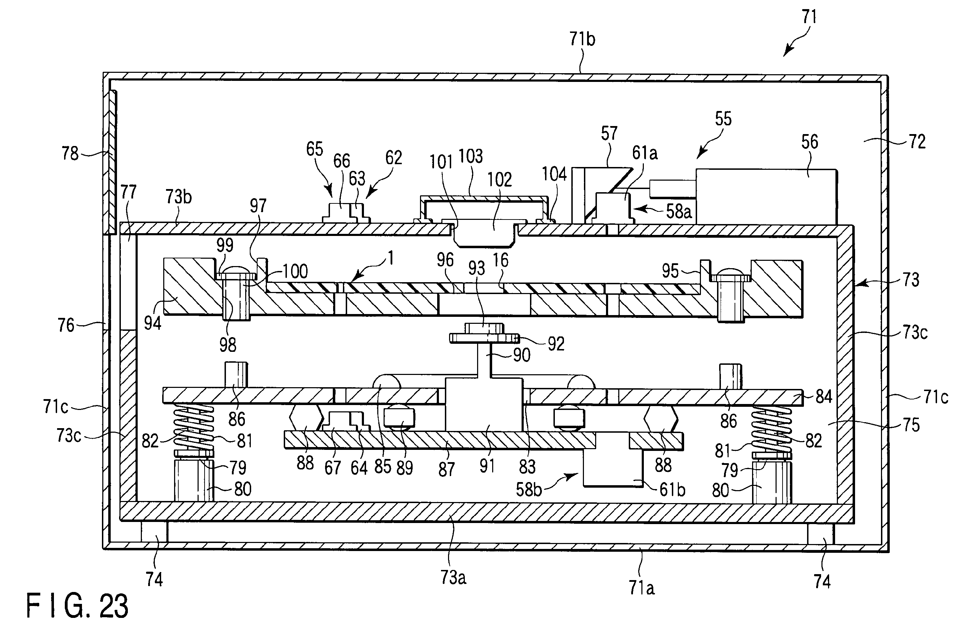

Prepared is the clinical examination disk 1, which has the cells 3 and 4 each having the specimen reservoirs, the reaction tanks, and the first and second narrow tubes (each not illustrated) having at the center the supporting hole 16. A gelatinized reagent is beforehand held in each of the reaction tanks. A specimen (for example, a blood) is injected into the specimen reservoir in each of the cells 3 and 4 of the disk 1. The shutter 78 is used to open the passage holes 76 and 77. As illustrated in FIG. 19, the tray 94 is then taken out from the outer packaging member 71, and the disk 1 is put onto the circular concave 95 thereof. As illustrated in FIG. 22, the pistons 79 of the cylinders 80 of the linearly-acting unit are lowered. As described above, according to this method, the motor-placing plate 87 is lowered together with the base plate 84, and the motor 91 fixed on the motor-placing plate 87 is lowered so that the disk-placing circular plate 92 on the upper end of the rotating axis 90 thereof and the columnar body 93 on the disk-placing circular plate 92 are also lowered. By this operation, the rotating axis 90 of the motor 91, the disk-placing circular plate 92, and the columnar body 93 are retreated from a region into which the tray 94 is carried, so that the region becomes able to receive the tray 94. Through the loading/unloading mechanism (not illustrated), the tray 94 is taken through the passage holes 76 and 77 into the chamber 75 region between the base plate 84 and the upper wall 73b of the case 73. When the taking-in of the tray 94 is finished, the supporting hole 16 in the disk 1 put on the tray 94 is positioned just above the columnar body 93 connected to the motor 91 as illustrated in FIG. 23.

The shutter 78 is used to close the passage holes 76 and 77, and then the pistons 79 of the cylinders 80 of the linearly-acting unit are raised. As descried above, the motor-placing plate 87 is raised together with the base plate 84. By the raise of the motor-placing plate 87, the motor 91 on the motor-placing plate 87 is raised as illustrated in FIG. 19, so that the disk-placing circular plate 92 on the upper end of the rotating axis 90 is passed through the hole 96 made open at the center of the circular concave 95 in the tray 94. The disk-placing circular plate 92 is brought into contact with the lower surface of the disk 1 put on the tray 94, so as to push up the disk 1, and further the columnar body 93 of the disk-placing circular plate 92 is inserted into the supporting hole 16 in the disk 1. The disk-placing circular plate 92 is further raised so that the disk 1 is brought into contact with the lower surface of the magnetic clamper 102 inserted in the hole 101 in the upper wall 73b of the case 73. Following this, the clamper 102 is magnetically coupled with the columnar body 93 by the magnetic force thereof. In other words, the disk 1 is sandwiched between the magnetic clamper 102 and the disk-placing circular plate 92, which are positioned above and below, respectively, so as to be fixed onto the disk-placing circular plate 92. Simultaneously, following the raise of the base plate 84, the pins 86 thereon are brought into contact with the lower surface of the third ringed packing 100 inserted in the ringed hole 98 in the tray 94 to be vertically movable. Following this, the third ringed packing 100 is held upward so that the upper end thereof is brought into contact with the inner surface of the upper wall 73b of the case 73. Following the raise of the base plate 84, the first ringed packing 85 inside the pins 86 on the base plate 84 is brought into contact with the lower surface of the tray 94.

By this operation, the disk 1 is set onto the disk-placing circular plate 92 of the rotating axis 90 of the motor 91. After the disk 1 is set, an electric current is passed through the film heater 107 of the first thin-film heater unit 105. Heat from the film heater 107 is transmitted through the heat radiating sheet 106 to the tray 94 made of aluminum so that the disk 1, positioned in the chamber 75 region between the tray 94 and the upper wall 73b, is heated from below. Simultaneously, an electrical current is passed through the film heater 112 of the second thin-film heater unit 111 on the inner surface of the upper wall 73b of the case 73. Heat from the film heater 112 is transmitted through the heat radiating sheet 113 and the aluminum plate 114 to heat the disk 1, positioned in the chamber 75 region between the tray 94 and the upper wall 73b of the case 73, from above.

After the setting of the disk 1 onto the disk-placing circular plate 92 and the heating of the disk 1 by effect of the first and second thin-film heater units 105 and 111, the rotating axis 90 of the motor 91 is rotated at a high speed. At this time, the disk 1 sandwiched between the disk-placing circular plate 92 of the rotating axis 90 and the magnetic clamper 102 is rotated at a high speed so that blood plasma separated by the centrifugation as described above, in the cell 3 of the disk 1, is sent into the reaction tank thereof. Thus, the blood plasma is caused to react with the liquefied reagent therein. Thereafter, in a manner equivalent to that for analyzing the blood by use of the disk, the concentration of, for example, a target substance in the blood plasma is measured by use of the excited-light-radiating unit 55, the paired fluorescence receiving units 58a and 58b, the disk-rotation-number-detecting photo-interrupter 62, and the timing-detecting photo-interrupter 65.

In the clinical examination device having this structure, the upper end of the third ringed packing 100 pushed up by the pins 86 is brought into contact with the inner surface of the upper wall 73b of the case 73, thereby causing the air to flow to the outermost region with the high-speed rotation of the disk 1. Thus, the gap between the upper wall 73b of the case 73 and the tray 94, in which air is to be taken in from the outside, can be shut out. That is to say, the chamber 75 region between the tray 94 and the upper wall 73b of the case 73, which is a space for arranging the disk 1, can be made into an airtight state. As a result, the generation of air flow in the chamber 75 region, which follows the high-speed rotation of the disk 1, is restrained so that the cooling of the disk 1 can be restrained. Therefore, it becomes possible to control precisely the temperature of the reaction between the blood plasma and the reagent in the reaction tank of the cell 3 of the disk 1 heated by effect of the first and second thin-film heater units 105 and 111. The precise control of the reaction temperature makes it possible that the gelatinized reagent in the reagent reservoir formed in the cell 3 of the disk 1 is evenly liquefied before the reaction.

The first ringed packing 85 is arranged on the base plate 84 and inside the positions of the pins 86. When the base plate 84 is raised, the first ringed packing 85 is brought into contact with the lower surface of the tray 94. Thus, the gap between the base plate 84 and the tray 94 is blocked with the first ringed packing 85. This makes it possible to keep, at a higher-level airtight state, the chamber 75 region, which is a space for arranging the disk 1, between the tray 94 and the upper wall 73b of the case 73. As a result, at the time of rotating the disk 1 at a high speed, a more precise control can be attained over the temperature of the reaction between the blood plasma and the reagent in the reaction tank of the cell 3 of the disk 1.

The second ringed packing 89 is interposed between the base plate 84 and the motor-placing plate 87 to block the gap between the base plate 84 and the motor-placing plate 87. It is therefore possible to keep, at a higher-level airtight state, the chamber 75 region, which is a space for arranging the disk 1, between the tray 94 and the upper wall 73b of the case 73. As a result, at the time of rotating the disk 1 at a high speed, a still more precise control can be attained over the temperature of the reaction between the blood plasma and the reagent in the reaction tank of the cell 3 of the disk 1.

In the structure wherein the disk 1 is sandwiched between the disk-placing circular plate 92 and the magnetic clamper 102, the upper-region-closing cylindrical cover 103 is fixed onto the outer surface of the upper wall 73b of the case 73 with the fourth disk-form packing 104 interposed therebetween, so as to cover the clamper 102. Thus, the gap between the clamper 102 and the hole 29 in the upper wall 73b of the case 73 is blocked from the outside. It is therefore possible to keep, at a higher-level airtight state, the chamber 75 region, which is a space for arranging the disk 1, between the tray 94 and the upper wall 73b of the case 73. As a result, at the time of rotating the disk 1 at a high speed, a still more precise control can be attained over the temperature of the reaction between the blood plasma and the reagent in the reaction tank of the cell 3 of the disk 1.

Accordingly, the clinical examination device according to the embodiment makes it possible to control the temperature of the reaction between the blood plasma and the reagent precisely in the cell 3 of the disk 1 rotated at a high speed. Thus, a blood examination high in reproducibility can be attained.

The third ringed packing 100 is inserted into the ringed hole 98 in the tray 94 to be vertically movable; thus, the third ringed packing 100 is pushed up by the pins 86 on the raised base plate 84 so as to be brought into contact with the inner surface of the upper wall 73b of the case 73 only when the disk 1 is rotated at a high speed. When not rotated, in the step of taking in or taking out the tray 94, the third ringed packing 100 is lowered as illustrated in FIGS. 23 and 24, so that a predetermined distance is kept between the packing 100 and the upper wall 73b of the case 73. In short, the upper end of the third ringed packing 100 does not contact the inner surface of the upper wall 73b. As a result, when the tray 94 is taken in or taken out by means of the loading/unloading mechanism, the upper end of the third ringed packing 100 can be prevented from being slid on the upper wall 73b to be worn off in a short period.

The clinical examination device according to the embodiment is made into a structure wherein the third ringed packing fitted to the tray is pushed up by the pins arranged vertically in the base plate. It is allowable that instead of the pins, for example, a ringed projection is attached onto the base plate and the third ringed packing of the tray is pushed up by this ringed projection. The ringed projection functions as a member for pushing the third ringed packing, and further makes it possible to block the gap between the base plate and the tray. As a result, it is possible to omit the first ringed packing set on the base plate to block the gap between the base plate and the tray.

In the clinical examination device according to the embodiment, the thin-film heater units for heating the disk are arranged onto the tray lower surface and the upper wall inner surface of the case, respectively. However, a thin-film heater unit may be arranged onto any one of the tray lower surface and the upper wall inner surface of the case. In other words, the disk is made of a thin synthetic resin, so that the whole of the disk can be heated when a thin-film heater unit is arranged onto any one of the tray lower surface and the upper wall inner surface of the case.

In the clinical examination device according to the embodiment, the linearly-acting unit is not limited to a structure including cylinders having four pistons which are each independently vertically movable. For example, it is allowable to fix, onto the inner surface of the bottom plate of the case, two linearly-acting mechanisms each provided with a slider having a cam groove, a lifting-up blanket to which a cam follower engaged with a cam groove is horizontally linked, and two pistons located vertically onto this blanket. Explanation of Reference Symbols
- 1: Clinical examination disk
- 2: Disk body
- 3, 4: Cell
- 5, 6: Window plate
- 7: Reservoir
- 9: Specimen injecting port
- 10: Reaction tank
- 12: First narrow tube
- 14: Second narrow tub
- 17: Rotation-number-detecting hole
- 18₁, 18₂: Timing detecting hole
- 51: Rotationally driving mechanism
- 62: Disk-rotation-number-detecting photo-interrupter
- 65: Timing-detecting photo-interrupter
- 21, 28: Overflow tank
- 22: Third narrow tub
- 23: Buffer tank
- 24: Air trap
- 27: Reagent reservoir
- 28: Peeling mount
- 29: Adhesive tape
- 30, 31: circular rib
- 37, 38: Rectangular groove
- 41: Bag
- 42: Small bag
- 44: Humidity adjusting agent
- 71: Outer packaging member
- 73: Case
- 73b: Upper wall
- 75: Chamber
- 80: Cylinder
- 84: Base plate
- 85: First disk-form packing 100
- 86: Pin
- 87: Motor-placing plate
- 89: Second ringed packing
- 91: Motor
- 92: Disk-placing circular plate
- 93: Metal columnar body
- 94: Tray
- 95: Circular concave
- 89: Third ringed packing
- 102: Clamper
- 103: Upper-region-closing cylindrical cover
- 104: Fourth disk-form packing
- 105, 111: First thin-film heater unit
- 107, 112: Film

## Claims

1. A clinical examination disk, comprising a disk body in a disk form, and at least one cell formed in the disk body,
wherein the cell comprises:
a specimen reservoir formed in the disk body and having a specimen injecting port;
a reaction tank formed in the disk body so as to position nearer to the outer circumference of the body than a position of the specimen reservoir, and holding a reagent which is reactive with the specimen and is beforehand gelatinized;
a first narrow tube formed in the disk body, and communicating to the specimen reservoir and the reaction tank; and
a second narrow tube formed in the disk body, and having one end connected to the reaction tank and the other end made open.

2. The clinical examination disk according to claim 1, wherein the gelatinized reagent is obtained by introducing a reagent in which a gelatinizer is added into the reaction tank, and gelatinizing the agent at a refrigerating temperature.

3. The clinical examination disk according to claim 2, wherein the gelatinizer is at least one selected from the group consisting of gelatin, sodium alginate, carrageenan and pectin.

4. The clinical examination disk according to claim 1, wherein the cells are two or more cells formed in the disk body, and the specimen reservoir in at least one of these cells holds a standard sample for calibration.

5. The clinical examination disk according to claim 1, wherein the cell further comprises a first overflow tank formed in the disk body so as to position nearer to the outer circumference of the disk body than the specimen reservoir, and a third narrow tube branched from the first narrow tube and having a tip connected to the first overflow tank.

6. The clinical examination disk according to claim 1, wherein the cell further comprises a buffer tank and an air trap formed in the disk body between the specimen reservoir and the reaction tank, and a fourth narrow tube formed in the disk body, one end of the tube being connected to the buffer tank, and the other end thereof being made open,
the buffer tank and the air trap are arranged in the order described herein from the vicinity of the specimen reservoir toward the reaction tank, and
the specimen reservoir is connected to the reaction tank by passing the first narrow tube from the specimen reservoir through a position between the buffer tank and the air tap, and then through a position between the air trap and the reaction tank.

7. The clinical examination disk according to claim 5, wherein the cell further comprises:
a second overflow tank which is formed in the disk body so as to adjoin to along the circumferential direction of the disk body from the specimen reservoir as a starting point;
a fifth narrow tube having one end connected to the specimen reservoir so as to position at a position which is nearer to the center of the disk body than the connection position of the first narrow tube connected to the specimen reservoir, and having the other end connected to the second overflow tank; and
a sixth narrow tube having one end extended to the second overflow tank and the other end extended to the disk body outside the cell.

8. The clinical examination disk according to claim 1, wherein the specimen reservoir and the reaction tank constitutes a reservoir-forming hole and a reaction-tank-forming hole which are made open in the disk body, respectively, a first window plate formed onto the front surface of the disk body so as to cover the reservoir-forming hole and the reaction-tank-forming hole, and a second window plate formed onto the rear surface of the disk body so as to cover the reservoir-forming hole and the reaction-tank-forming hole,
wherein the first and second window plates are melt-bonded to a circular rib projected from the front and rear surfaces of the disk body, and the circular rib is projected at least from front and rear surface regions of the disk body around the reaction-tank-forming hole so as to be integrated with the surface regions.

9. The clinical examination disk according to claim 1, wherein the cell further comprises a reagent reservoir formed in the disk body so as to position nearer to the center of the disk than the reaction tank, and a fifth narrow tube formed in the disk body in order to connect the reaction tank and the reagent reservoir to each other,
wherein before an examination, a reagent added a gelatinizer in the reagent reservoir is introduced through the fifth narrow tube into the reaction tank, and the reagent is gelatinized at a refrigerating temperature so that the reagent gelatinized beforehand is held in the reaction tank.

10. The clinical examination disk according to claim 1, wherein the cell further comprises a peeling mount arranged to cover the specimen injecting port in the specimen reservoir, and an adhesive tape bonded so as to extend from the peeling mount to the disk body and configured to fix the peeling mount to the disk body.

11. The clinical examination disk according to claim 1, wherein the cell is arranged to be attachable to the disk body and detachable therefrom.

12. A disk pack, comprising a moisture-proof bag, a clinical examination disk as recited in claim 1 that is air-tightly held in the bag, and a moisture-adjusting agent held, together with the clinical examination disk, in the bag.

13. A clinical examination device, comprising:
an outer packaging member;
a case arranged in the outer packaging member and having a chamber inside the case;
a linearly-acting unit comprising a base plate arranged in the chamber of the case to be vertically movable;
a passage hole made open a portion which is extended from an upside portion of a side wall of the case to a side wall of the outer packaging member;
a tray which is capable of putting into the chamber through the passage hole and taking out the chamber through the passage hole, and having a circular concave onto which a clinical examination disk as recited in claim 1 is placed; and
a rotating unit fixed to the linearly-acting unit to cause the disk to be rotated,
wherein the tray comprises a ringed hole made open in a ringed region around the outer circumference of the circular concave, and a ringed packing fixed to the ringed hole to be vertically movable,
the base plate comprises, on an upper surface thereof, a pushing-up member to push up the ringed packing, and
a thin-film heater unit is formed on at least one of the inner surface of an upper wall of the case and a lower surface of the tray.

14. The clinical examination device according to claim 13, wherein the pushing-up member in which the base plate forms on the upper surface thereof is a ringed projection.

15. The clinical examination device according to claim 13, wherein the pushing-up member in which the base plate forms on the upper surface thereof is a plurality of pins.

16. The clinical examination device according to claim 13, wherein the rotating means comprises a motor having a rotating axis, and a disk-placing plate attached to the upper end of the rotating axis.

17. The clinical examination device according to claim 13, which further comprises another ringed packing formed inside the pushing-up member on the base plate.

18. The clinical examination device according to claim 16, which further comprises a clamper set to the upper wall of the case so as to hold the disk accompanied with the disk-placing plate of the motor, a cover attached to the upper wall so as to cover the clamper, and a packing interposed between the clamper and the cover.

19. The clinical examination device according to claim 16, wherein the linearly-acting unit further comprises a motor-placing plate hung under the base plate to fix the motor, and another ringed packing is interposed between the motor-placing plate and the base plate such as to surround the motor.
